# EUROPEAN PATENT SPECIFICATION

(11) **EP 3 455 199 B1**
(45) Date of publication and mention of the grant of the patent: **13.05.2020**
(21) Application number: 17721752.8
(22) Date of filing: 11.05.2017
(51) Int. Cl.: C07C 29/149, C07C 31/20, C07C 31/125, C07C 33/14, C07C 33/22, C07C 33/32, B01J 31/02, B01J 31/22, B01J 31/24

(54) **SELECTIVE REDUCTION OF ESTERS TO ALCOHOLS**
SELEKTIVE REDUKTION VON ESTERN ZU ALKOHOLEN
RÉDUCTION SÉLECTIVE D'ESTERS EN ALCOOLS

(30) Priority: 13.05.2016 WO PCT/EP2016/169509; 23.01.2017 WO PCT/EP2017/152592
(43) Date of publication of application: 20.03.2019
(73) Proprietor: DSM IP Assets B.V., 6411 TE Heerlen (NL)
(72) Inventor: BELLER, Matthias, 18059 Rostock (DE); BONRATH, Werner, 4303 Kaiseraugst (CH); DE VRIES, Johannes, Gerardus, 18059 Rostock (DE); FAN, Yuting, 18059 Rostock (DE); HÜBNER, Sandra, 18059 Rostock (DE); LEFORT, Laurent, 4303 Kaiseraugst (CH); MEDLOCK, Jonathan, Alan, 4303 Kaiseraugst (CH); PUYLAERT, Pim, 18059 Rostock (DE); VAN HECK, Richard, 18059 Rostock (DE)
(74) Representative: Kurt, Manfred
(86) International application number: PCT/EP2017/061299
(87) International publication number: WO 2017/194663

(56) References cited:
- SVENJA WERKMEISTER ET AL: "Catalytic Hydrogenation of Carboxylic Acid Esters, Amides, and Nitriles with Homogeneous Catalysts", ORGANIC PROCESS RESEARCH AND DEVELOPMENT, vol. 18, no. 2, 13 January 2014 (2014-01-13), pages 289-302, XP055311022, US ISSN: 1083-6160, DOI: 10.1021/op4003278
- SUJAN BISWAS ET AL: "Rhodium(III)-triphenylphosphine complex with NNS donor thioether containing Schiff base ligand: Synthesis, spectra, electrochemistry and catalytic activity", JOURNAL OF MOLECULAR STRUCTURE., vol. 1099, 29 June 2015 (2015-06-29), pages 297-303, XP055310912, NL ISSN: 0022-2860, DOI: 10.1016/j.molstruc.2015.06.065

## Description

The present invention relates to a selective reduction of esters to their corresponding alcohols.

Reduction of an ester into the corresponding alcohol is a fundamental and very important reaction in organic chemistry, and is used in a large number of chemical processes. The obtained alcohols are used as such or are important intermediates in further chemical processes.

To reduce such esters, usually harsh reaction conditions have to be applied. Furthermore, the reduction of esters usually requests the use of highly reactive reducing agents such as LiAlH₄ or NaBH₄, which are not easy to handle and which produce a lot waste as a result of the reaction.

The review article Werkmeister et al., Org. Process Res. Dev. 2014, 18, 289-302, discloses complexes which have been found to catalyse the hydrogenation of esters to alcohols.

The esters which are reduced in the context of the present invention are esters of formula (I) 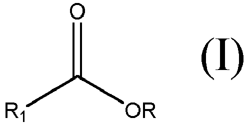 wherein R is a linear C₁-C₆-alkyl group, which can be substituted; a branched C₃-C₆-alkyl group, which can be substituted or a benzyl group, which can be substituted, and
R₁ can be a suitable organic moiety (which is defined below).

The goal of the present invention was to provide a process for the improved production of the following compounds of formula (II)

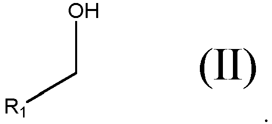

As stated above the esters, which are of interest in the context of the present patent application are those of formula (I) 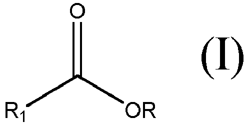 wherein
- R: is a linear C₁-C₆-alkyl group, which can be substituted; a branched C₃-C₆-alkyl group, which can be substituted or a benzyl group, which can be substituted, and
- R₁: is an aromatic ring system which is unsubstituted (such as a benzene ring) or an aromatic ring system which is substituted; a heteroaromatic ring system which is unsubstituted or a heteroaromatic ring system which is substituted; an aliphatic ring system which is unsubstituted or an aliphatic ring system which is substituted; -CH₃; -CH₂CH₃; an unsubstituted C₃ - C₂₂ alkyl group, which can be linear or branched and which can also be partially unsaturated (comprising C-C double bond(s)); or an substituted C₂ - C₂₂ alkyl group, which can be linear or branched and which can also be partially unsaturated,
or R and R₁ form together a 4 to 7 membered ring system, which can be substituted.

The corresponding alcohols, which are the selectively hydrogenated products are those of formula (II) 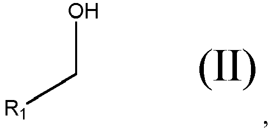 wherein the substituent R₁ has the same definition as in formula (I).

Surprisingly it was found that by the use of new specific catalysts, it is possible to selectively reduce the compounds of formula (I) in excellent yield and selectivity under mild reaction conditions.

The catalysts, which are used in the selective reduction (hydrogenation) according to the present invention are transition metal catalysts of formula (III)

[M(L)(X)ₐ(L')_{b}] **(III)**,

wherein
M is a transition metal (preferably a transition metal chosen from the group consisting of Os, Co, Ru and Fe, more preferably from the group consisting of Ru and Fe) and
X is an anion(preferably a halogen anion, a carboxylate (such as acetate or benzoate), borohydride (such as BH₄⁻), hydride, BF₄⁻, or PF₆⁻, more preferably a halogen anion, most preferably Cl⁻), and
L' is a monodentate ligand (preferably a monodentate phosphine ligand, more preferably triphenylphosphine (=PPh₃)), and
L is a tridentate ligand (which means that the ligand can be bound to the M at up to three sites) of formula (IV) 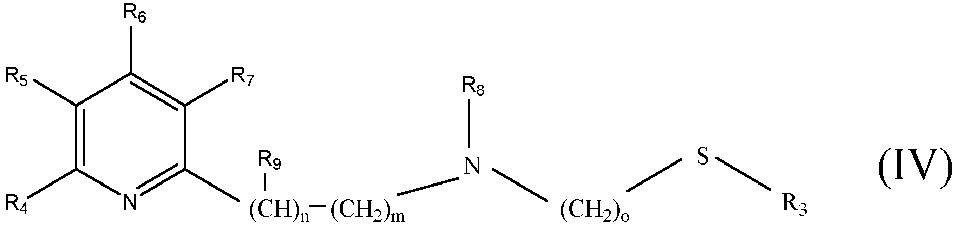 wherein
R₃ is a linear C₁ - C₄ alkyl group, which can be substituted; a branched C₃ - C₄ alkyl group, which can be substituted; or a phenyl group, which can be substituted, and
R₄ is H; a linear C₁ - C₄ alkyl group, which can be substituted; a branched C₃ - C₄ alkyl group, which can be substituted; or OC₁-C₂alkyl, and
R₅ is H; a linear C₁ - C₄ alkyl group, which can be substituted; a branched C₃ - C₄ alkyl group, which can be substituted; or OC₁-C₂alkyl,
or R₄ and R₅ form a C₄-C₈ ring system, which can be aliphatic or aromatic, and
R₆ is H; a linear C₁ - C₄ alkyl group, which can be substituted; a branched C₃ - C₄ alkyl group, which can be substituted;or OC₁-C₂alkyl, and
R₇ is H; a linear C₁ - C₄ alkyl group, which can be substituted; a branched C₃ - C₄ alkyl group, which can be substituted;or OC₁-C₂alkyl, and
R₈ is H or a a linear C₁ - C₄ alkyl group, which can be substituted; a branched C₃ - C₄ alkyl group, which can be substituted, and
R₉ is -CH₃ or -CH₂CH₃, and
m is 0, 1 or 2, and
n is 0, 1 or 2,
with the proviso that the sum of m+n is 1 or 2,
o is 2 or 3,
a is 0, 1, 2, or 3,
b is 0, 1, 2, or 3,
with the proviso that the sum of a+b is 2, 3 or 4.

From the state of the art, it is known that transition metal complexes can exist as monomers as well as dimers or even oligomers. The present formula (III) defines the empirical formula of the catalyst.

Therefore the present invention relates to a process (P) of production of a compound of formula (II) 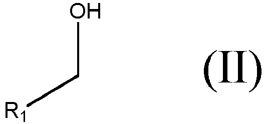 wherein
- R₁: is an aromatic ring system which is unsubstituted (such as a benzene ring) or an aromatic ring system which is substituted; a heteroaromatic ring system which is unsubstituted or a heteroaromatic ring system which is substituted; an aliphatic ring system which is unsubstituted or an aliphatic ring system which is substituted; -CH₃; -CH₂CH₃; an unsubstituted C₃ - C₂₂ alkyl group, which can be linear or branched and which can also be partially unsaturated (comprising C-C double bond(s)); or an substituted C₂ - C₂₂ alkyl group,
which can be linear or branched and which can also be partially unsaturated by a selective reduction of a compound of formula (I) 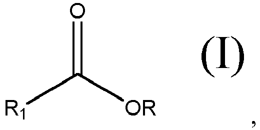 wherein
- R: is a linear C₁-C₆-alkyl group, which can be substituted; a branched C₃-C₆-alkyl group, which can be substituted or a benzyl group, which can be substituted, and
- R₁: is an aromatic ring system which is unsubstituted (such as a benzene ring) or an aromatic ring system which is substituted; a heteroaromatic ring system which is unsubstituted or a heteroaromatic ring system which is substituted; an aliphatic ring system which is unsubstituted or an aliphatic ring system which is substituted; -CH₃; -CH₂CH₃; an unsubstituted C₃ - C₂₂ alkyl group, which can be linear or branched and which can also be partially unsaturated (comprising C-C double bond(s)); or an substituted C₂ - C₂₂ alkyl group, which can be linear or branched and which can also be partially unsaturated,
or R and R₁ form together a 4 to 7 membered ring system, which can be substituted, characterised in that the selective reduction is carried out in the presence of at least one transition metal catalyst of formula (III)

[M(L)(X)ₐ(L')_{b}] **(III)**,

wherein
M is a transition metal and
X is an anion, and
L' is a monodentate ligand, and
L is a tridentate ligand of formula (IV) 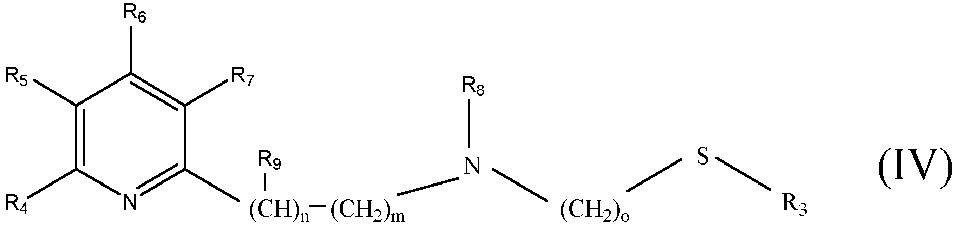 wherein
   R₃ is a linear C₁ - C₄ alkyl group, which can be substituted; a branched C₃ - C₄ alkyl group, which can be substituted; or a phenyl group, which can be substituted, and
   R₄ is H; a linear C₁ - C₄ alkyl group, which can be substituted; a branched C₃ - C₄ alkyl group, which can be substituted; or OC₁-C₂alkyl, and
   R₅ is H; a linear C₁ - C₄ alkyl group, which can be substituted; a branched C₃ - C₄ alkyl group, which can be substituted; or OC₁-C₂alkyl,
   or R₄ and R₅ form a C₄-C₈ ring system, which can be aliphatic or aromatic, and
   R₆ is H; a linear C₁ - C₄ alkyl group, which can be substituted; a branched C₃ - C₄ alkyl group, which can be substituted;or OC₁-C₂alkyl, and
   R₇ is H; a linear C₁ - C₄ alkyl group, which can be substituted; a branched C₃ - C₄ alkyl group, which can be substituted;or OC₁-C₂alkyl, and
   R₈ is H or a a linear C₁ - C₄ alkyl group, which can be substituted; a branched C₃ - C₄ alkyl group, which can be substituted, and
   R₉ is -CH₃ or -CH₂CH₃, and
   m is 0, 1 or 2, and
   n is 0, 1 or 2,
   with the proviso that the sum of m+n is 1 or 2,
   o is 2 or 3,
   a is 0, 1, 2, or 3,
   b is 0, 1, 2, or 3,
   with the proviso that the sum of a+b is 2, 3 or 4.

The process according to the present invention is preferably carried out in the presence of at least one base.

Preferably the base has the following formula (VIII)

M¹(OC₁-C₅alkyl) (VIII),

wherein M¹ is an alkali metal.

Preferred is a base of formula (VIII'),

M¹(OC₃-C₅alkyl) (VIII')

wherein
M¹ is Li, Na or K.

Especially preferred bases are selected from the group consisting of KOtBu, NaOtBu and LiOtBu.

Therefore the present invention relates to a process (P1), which is process (P), wherein the process is carried out in the presence of at least one base.

Therefore the present invention relates to a process (P1'), which is process (P1), wherein the process is carried out in the presence of at least one base of formula (VIII)

M¹(OC₁-C₅alkyl) (VIII),

wherein M¹ is an alkali metal.

Therefore the present invention relates to a process (P1"), which is process (P1), wherein the process is carried out in the presence of at least one base of formula (VIII'),

M¹(OC₃-C₅alkyl) (VIII')

wherein
M¹ is Li, Na or K.

Therefore the present invention relates to a process (P1'''), which is process (P1), wherein the process is carried out in the presence of at least one base selected from the group consisting of KOtBu, NaOtBu and LiOtBu.

The amount of the base can vary. Usually and preferably the base (or mixture of bases) is used in an amount of 0.1 - 5 mol-% (based on the number of moles of the compound of formula (I)).

Therefore the present invention relates to a process (P1""), which is process (P1), (P1'), (P1") or (P1'''), wherein 0.1 - 5 mol-% (based on the number of moles of the compound of formula (I)) of at least one base is used.

The catalyst which is used in the process of the present invention to selectively reduce the compound of formula (I) is a compound of formula (III) as defined above.

In a preferred embodiment the following catalysts are used:

[M(L)(X)ₐ(L')_{b}] **(III)**,

wherein
M is a transition metal chosen from the group consisting of Os, Co, Ru and Fe, and
X is a halogen anion, a carboxylate (such as acetate or benzoate), borohydride (such as BH₄⁻), hydride, BF₄⁻ or PF₆⁻, and
L' is a monodentate phosphine ligand, and
L is a tridentate ligand of formula (IV) 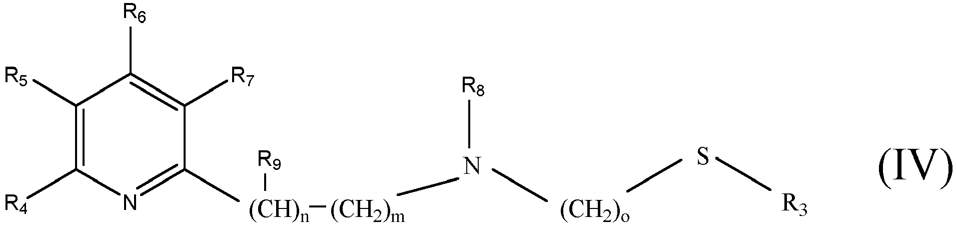
wherein
R₃ is -CH₃ or -CH₂CH₃, and
R₄ is H; -CH₃; -CH₂CH₃; -OCH₃ or -OCH₂CH₃, and
R₅ is H; -CH₃; -CH₂CH₃; -OCH₃ or -OCH₂CH₃,
or R₄ and R₅ form a C₄-C₈ ring system, which can be aliphatic or aromatic, and R₆ is H; -CH₃; -CH₂CH₃; -OCH₃ or -OCH₂CH₃, and
R₇ is H; -CH₃; -CH₂CH₃; -OCH₃ or -OCH₂CH₃, and
R₈ is H; -CH₃ or -CH₂CH₃, and
R₉ is -CH₃ or -CH₂CH₃, and
m is 0, 1 or 2, and
n is 0, 1 or 2,
with the proviso that the sum of m+n is 1 or 2,
o is 2 or 3,
a is 0, 1, 2, or 3,
b is 0, 1, 2, or 3,
with the proviso that the sum of a+b is 2 or 3.

In a more preferred embodiment the following catalysts are used:

[M(L)(X)ₐ(L')_{b}] **(III)**,

wherein
M is a transition metal chosen from the group consisting of Ru and Fe, and
X is a halogen anion (preferably Cl⁻), and
L' is triphenylphosphine, and
L is a tridentate ligand of formula (IV) 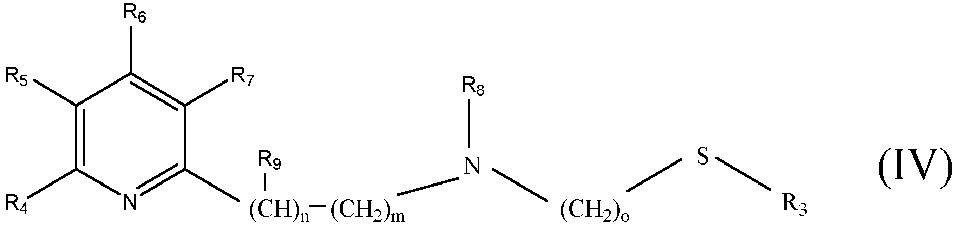
wherein
R₃ is -CH₃ or -CH₂CH₃, and
R₄ is H; -CH₃; -CH₂CH₃; -OCH₃ or -OCH₂CH₃, and
R₅ is H or -CH₃,
or R₄ and R₅ form a C₄-C₈ ring system, which can be aliphatic or aromatic, and
R₆ is H or -CH₃, and
R₇ is H or -CH₃, and
R₈ is H or -CH₃, and
R₉ is -CH₃, and
m is 0 or 1 and
n is 0 or 1,
with the proviso that the sum of m+n is 1,
o is 2,
a is 1 or 2,
b is 1 or 2,
with the proviso that the sum of a+b is 3.

In an especially preferred embodiment the following catalysts of formula (III')

M(L)(X)₂(L') **(III')**,

wherein
M is Ru or Fe, and
X is Cl⁻, and
L' is PPh₃, and
L is a tridentate ligand chosen from the group consisting of the ligands of formulae (IVa) - (IVI) 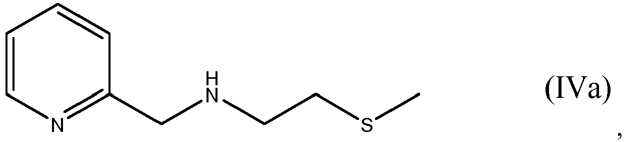 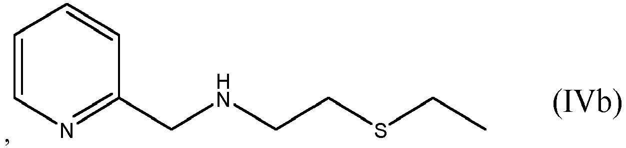 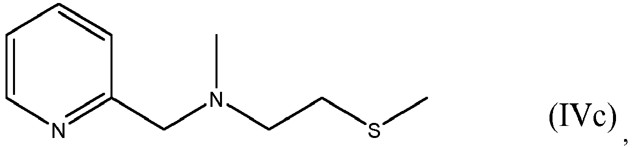 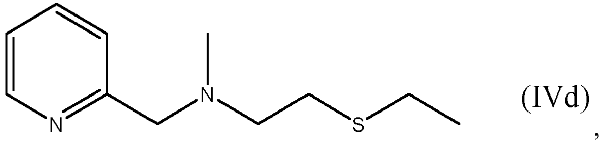 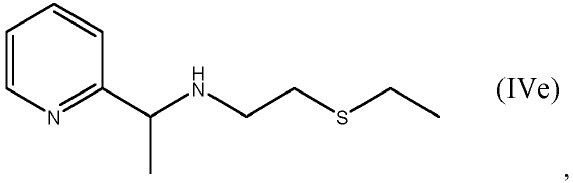 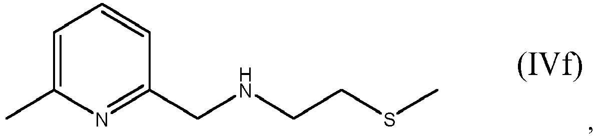 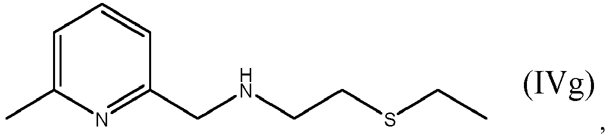 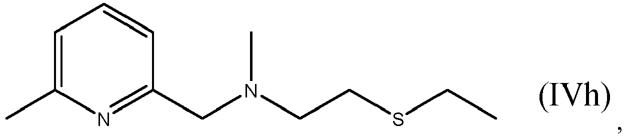 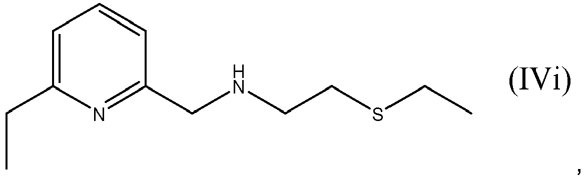 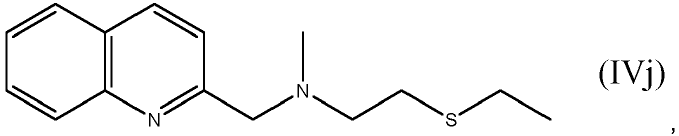 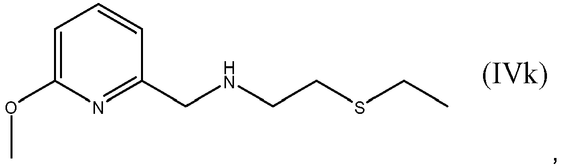 and 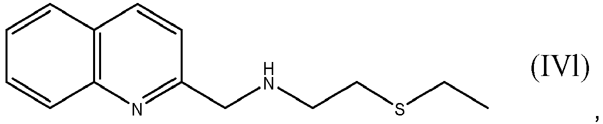 are used.

Therefore the present invention relates to a process (P2), which is process (P), (P1), (P1'), (P1"), (P1''') or (P1""), wherein the following catalysts of formula (III)

[M(L)(X)ₐ(L')_{b}] **(III)**,

wherein
M is a transition metal chosen from the group consisting of Os, Co, Ru and Fe, and
X is a halogen anion, a carboxylate (such as acetate or benzoate), borohydride
(such as BH₄⁻), hydride, BF₄⁻ or PF₆⁻, and
L' is a monodentate phosphine ligand, and
L is a tridentate ligand of formula (IV) 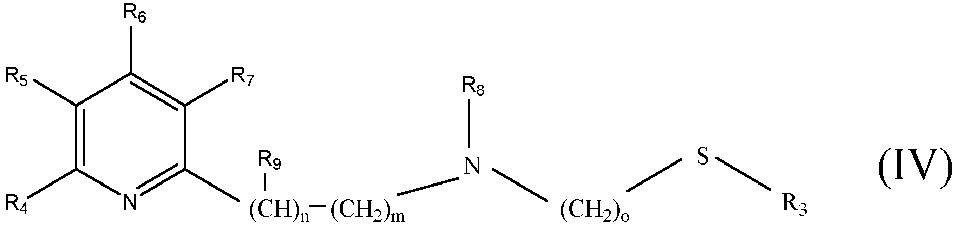 wherein
   R₃ is -CH₃ or -CH₂CH₃, and
   R₄ is H; -CH₃; -CH₂CH₃; -OCH₃ or -OCH₂CH₃, and
   R₅ is H; -CH₃; -CH₂CH₃; -OCH₃ or -OCH₂CH₃,
   or R₄ and R₅ form a C₄-C₈ ring system, which can be aliphatic or aromatic, and
   R₆ is H; -CH₃; -CH₂CH₃; -OCH₃ or -OCH₂CH₃, and
   R₇ is H; -CH₃; -CH₂CH₃; -OCH₃ or -OCH₂CH₃, and
   R₈ is H; -CH₃ or -CH₂CH₃, and
   R₉ is -CH₃ or -CH₂CH₃, and
   m is 0, 1 or 2, and
   n is 0, 1 or 2,
   with the proviso that the sum of m+n is 1 or 2,
   o is 2 or 3,
   a is 0, 1, 2, or 3,
   b is 0, 1, 2, or 3,
   with the proviso that the sum of a+b is 2 or 3,
   are used.

Therefore the present invention relates to a process (P2'), which is process (P), (P1), (P1'), (P1"), (P1''') or (P1""), wherein the following catalysts of formula (III)

[M(L)(X)ₐ(L')_{b}] **(III)**,

M is a transition metal chosen from the group consisting of Ru and Fe, and
X is a halogen anion (preferably Cl⁻), and
L' is triphenylphosphine, and
L is a tridentate ligand of formula (IV) 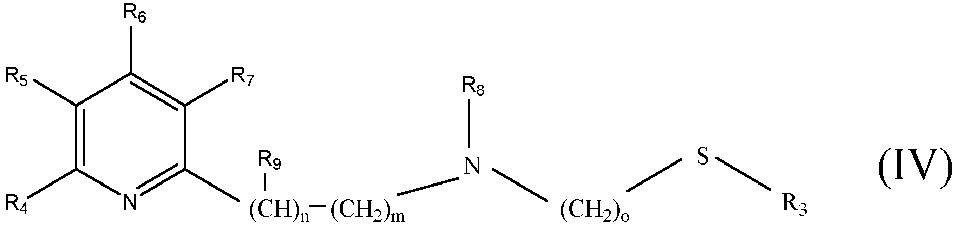 wherein
   R₃ is -CH₃ or -CH₂CH₃, and
   R₄ is H; -CH₃; -CH₂CH₃; -OCH₃ or -OCH₂CH₃, and
   R₅ is H or -CH₃, and
   or R₄ and R₅ form a C₄-C₈ ring system, which can be aliphatic or aromatic, and
   R₆ is H or -CH₃, and
   R₇ is H or -CH₃, and
   R₈ is H or -CH₃, and
   R₉ is -CH₃, and
   m is 0 or 1, and
   n is 0 or 1,
   with the proviso that the sum of m+n is 1,
   o is 2,
   a is 1 or 2,
   b is 1 or 2,
   with the proviso that the sum of a+b is 3,
   are used.

Therefore the present invention relates to a process (P2"), which is process (P), (P1), (P1'), (P1"), (P1''') or (P1""), wherein the following catalysts of formula (III')

M(L)(X)₂(L') **(III')**,

wherein
M is Ru or Fe, and
X is Cl⁻, and
L' is PPh₃, and
L is a tridentate ligand chosen from the group consisting of the ligands of formulae (IVa) - (IVI) 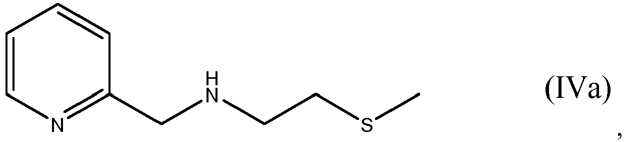 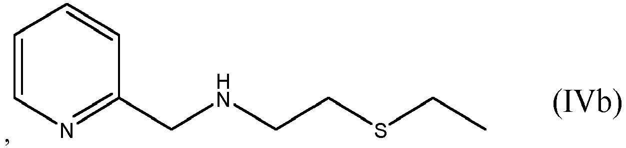 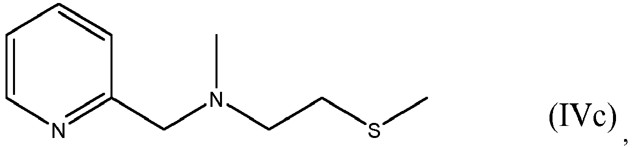 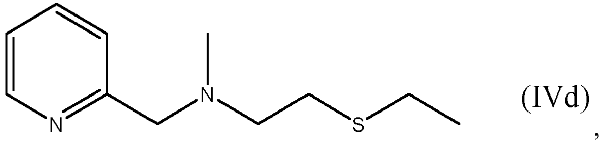 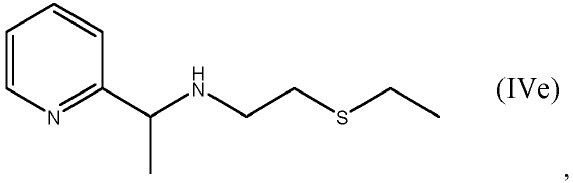 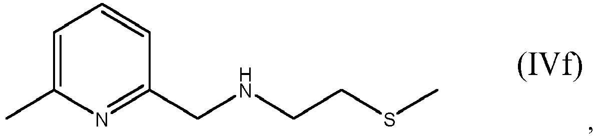 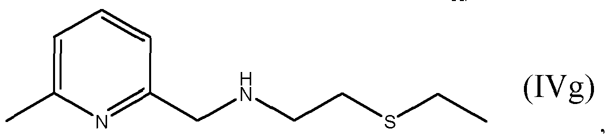 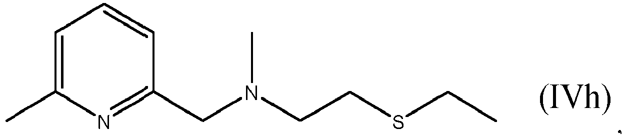 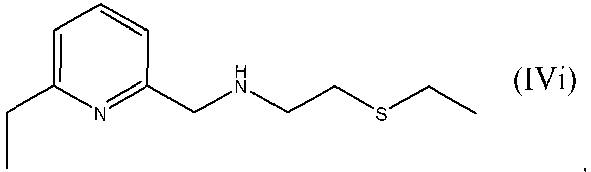 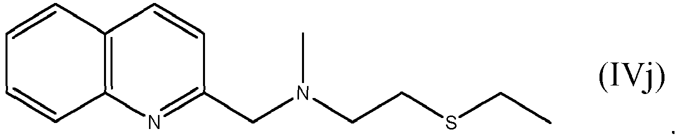 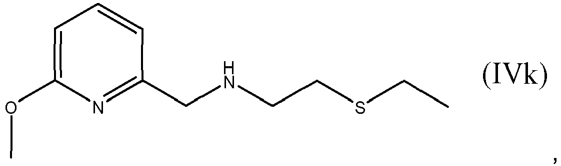 and 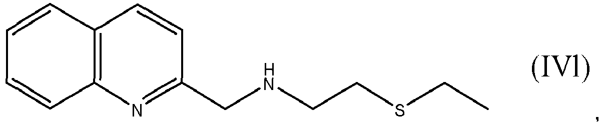 are used.

The catalysts which are used in the process of the present invention are also new. The synthesis of the catalyst are described in details below.

Preferred embodiments of the present invention relate to selective reductions of the following compounds of formula (I) 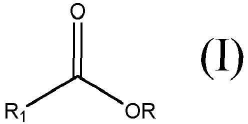
- R: is a linear C₁-C₄-alkyl group, which can be substituted; a branched C₃-C₆-alkyl group, which can be substituted or a benzyl group, which can be substituted
- R₁: is an unsubstituted benzene ring or benzene ring which is substituted; a heteroaromatic ring system which is unsubstituted or a heteroaromatic ring system which is substituted; an aliphatic ring system which is unsubstituted or an aliphatic ring system which is substituted; -CH₃; -CH₂CH₃; an unsubstituted C₂ - C₂₂ alkyl group, which can be linear or branched and which can also be partially unsaturated or a substituted C₂ - C₂₂ alkyl group, which can be linear or branched and which can also be partially unsaturated,
or R and R₁ form together a 4 to 7 membered ring system, which can be substituted.

More preferred embodiments of the present invention relate to selective reductions of the following compounds of formula (I) 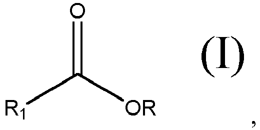
- R: is a linear C₁-C₄-alkyl group, which can be substituted or a branched C₃-C₆-alkyl group, which can be substituted
- R₁: is an unsubstituted benzene ring or benzene ring, which is substituted; a heteroaromatic ring system which is unsubstituted or a heteroaromatic ring system which is substituted; an aliphatic ring system which is unsubstituted; an aliphatic ring system which is substituted or a substituted C₂ - C₁₀ alkyl group, which can be linear or branched and which can also be partially unsaturated or a substituted C₂ - C₁₀ alkyl group which can also be partially unsaturated,
or R and R₁ form together a 4 to 7 membered ring system, which can be substituted

Especially preferred embodiments of the present invention relate to selective reductions of the following compounds of formula (Ia) to (If)

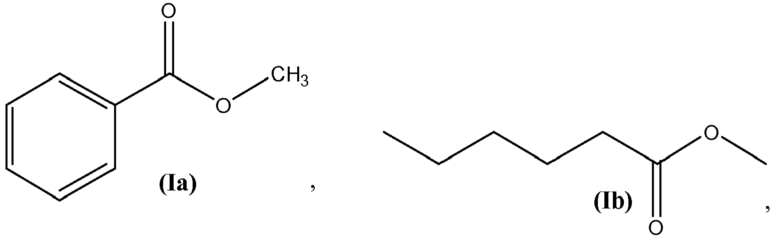

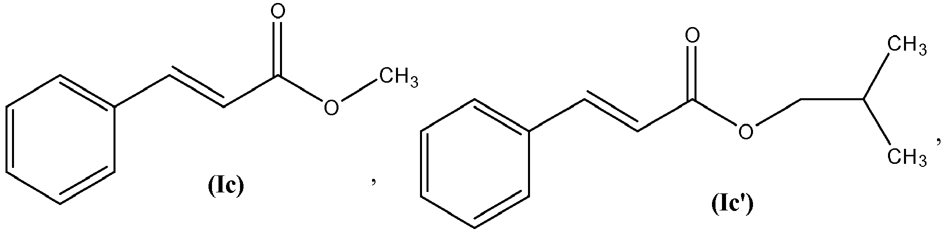

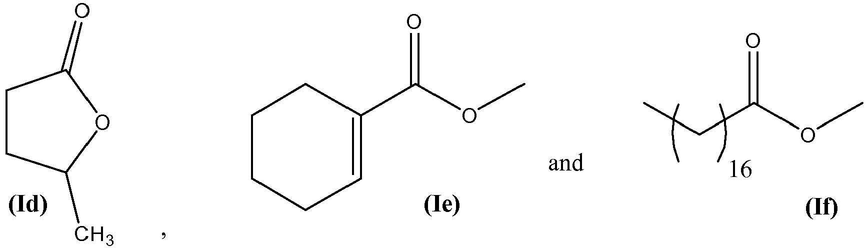

The following compounds of formulae (IIa) to (IIf) are the corresponding ones to the starting material (compounds of formula (Ia) to (If). The compounds of formula (Ic) and (Ic') do react to the same compound of formula (IIc):

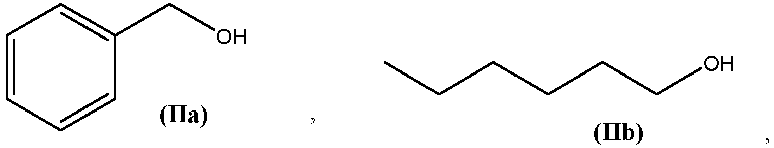

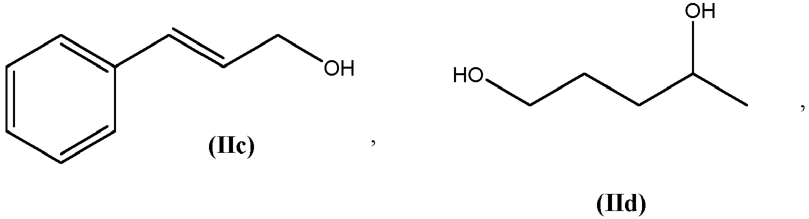

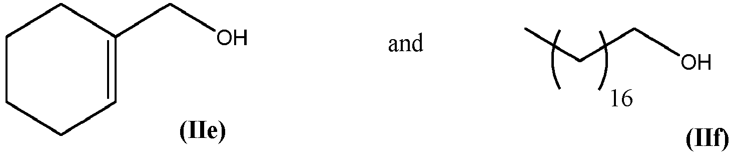

Therefore the present invention relates to a process (P3), which is process (P), (P1), (P1'), (P1"), (P1'''), (P1""), (P2), (P2') or (P2"), wherein a compound of formula (I) 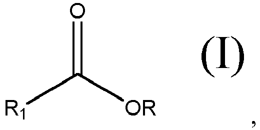
- R: is a linear C₁-C₄-alkyl group, which can be substituted; a branched C₃-C₆-alkyl group, which can be substituted or a benzyl group, which can be substituted
- R₁: is an unsubstituted benzene ring or benzene ring which is substituted; a heteroaromatic ring system which is unsubstituted or a heteroaromatic ring system which is substituted; an aliphatic ring system which is unsubstituted or an aliphatic ring system which is substituted; -CH₃; -CH₂CH₃; an unsubstituted C₂ - C₂₂ alkyl group, which can be linear or branched and which can also be partially unsaturated or a substituted C₂ - C₂₂ alkyl group, which can be linear or branched and which can also be partially unsaturated,
or R and R₁ form together a 4 to 7 membered ring system, which can be substituted, is selectively reduced.

Therefore the present invention relates to a process (P3'), which is process (P), (P1), (P1'), (P1"), (P1'''), (P1""), (P2), (P2') or (P2"), wherein a compound of formula (I) 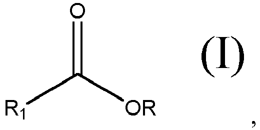
- R: is a linear C₁-C₄-alkyl group, which can be substituted or a branched C₃-C₆-alkyl group, which can be substituted
- R₁: is an unsubstituted benzene ring or benzene ring, which is substituted; a heteroaromatic ring system which is unsubstituted or a heteroaromatic ring system which is substituted; an aliphatic ring system which is unsubstituted; an aliphatic ring system which is substituted or a substituted C₂ - C₁₀ alkyl group, which can be linear or branched and which can also be partially unsaturated or a substituted C₂ - C₁₀ alkyl group which can also be partially unsaturated,
is selectively reduced.

Therefore the present invention relates to a process (P3"), which is process (P), (P1), (P1'), (P1"), (P1'''), (P1""), (P2), (P2') or (P2"), wherein a compound chosen from the group consisting of the following compounds 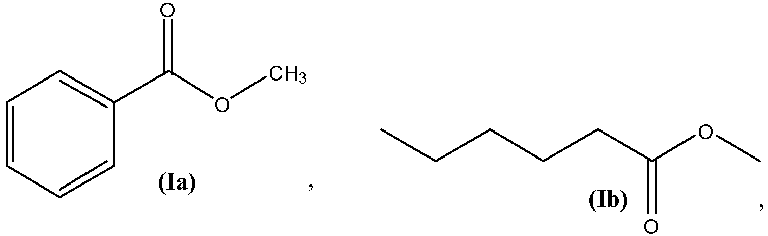 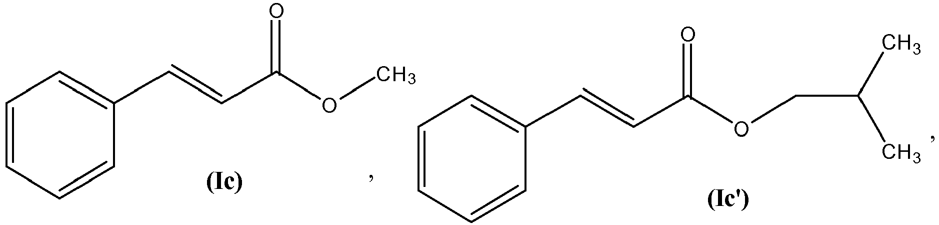 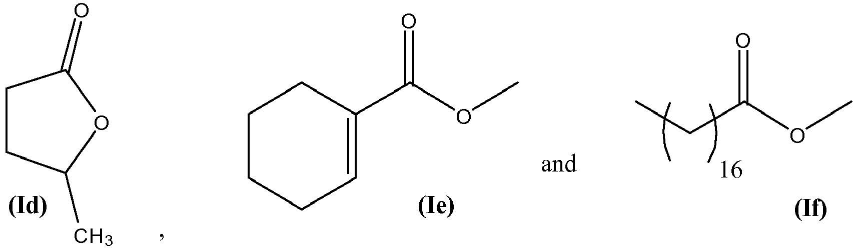 is selectively reduced.

In the following the synthesis of the catalyst used in the selective reduction of the present invention is described.

### Production of the ligand L (compounds of formula (IV))

The ligand (L) is usually made first and this ligand (L) is then used afterwards to synthesise the transition metal based catalyst of formula (III).

The production of the ligands (wherein R₈ is H) is usually done by the following reaction scheme (RS): 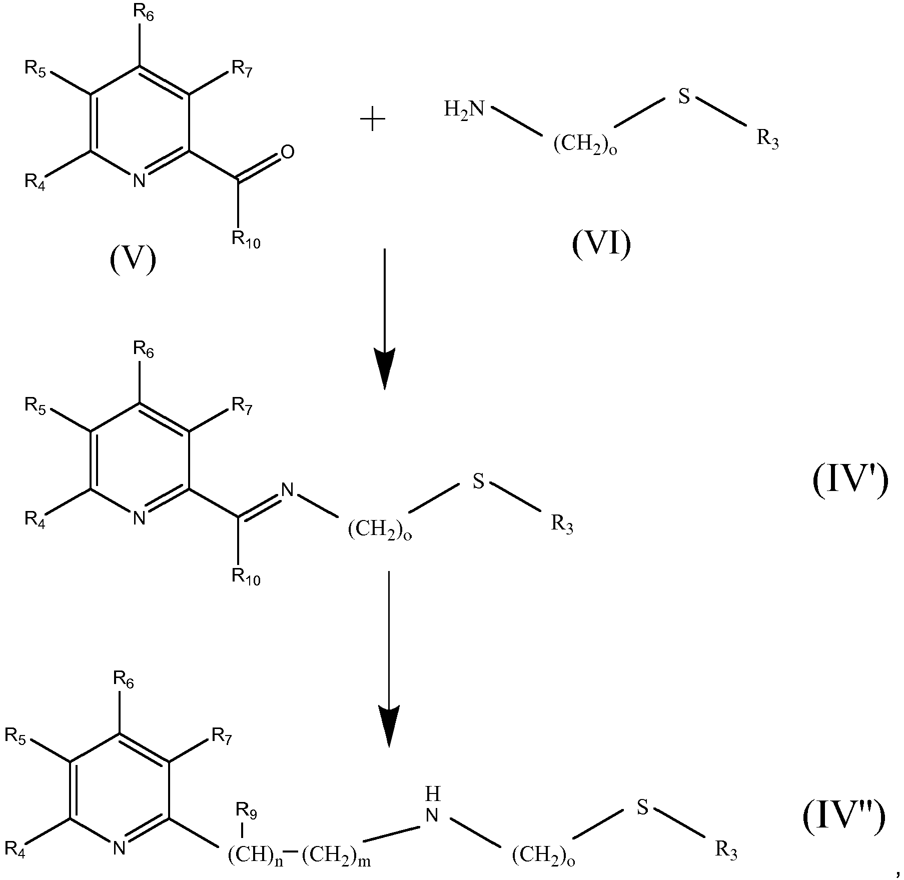 wherein R₁₀ is H or has the same meaning as Rg, all other substituents and letters have the meanings as defined above.

To obtain the ligands (wherein R₈ is -CH₃ or -CH₂CH₃), the process of RS is carried out and then in an additional step the amino group is alkylated.

The process of the production of the ligand is usually carried out in a solvent (or a mixture of solvents).

Suitable solvents are esters, ethers, amides, hydrocarbons, halogenated hydrocarbons and alcohols. Preferred solvents are CH₂Cl₂, toluene, ethyl acetate, THF, methanol and ethanol.

The process of the production of the ligand is usually carried out at temperature of between 0 and 120°C (preferably 0 - 40°C).

The process of the production of the ligand is usually carried at ambient pressure.

The obtained ligand of formula (IV") (with R₈ = H) is removed from the reaction mixture by extraction and can be further purified if required. The yield is very good.

To obtain the ligands of formula (IV) wherein R₈ is CH₃ or CH₂CH₃, the obtained ligand of formula (IV") is alkylated in an additional step.

This alkylation step can be carried out according to commonly known processes.

### Production of the catalyst (compounds of formula (III))

As stated above the catalysts which are used in the process of the present invention are new.

They are produced by commonly known processes. Usually (and preferably in the context of the present invention) they are produced as follows (reaction scheme (RS2)): 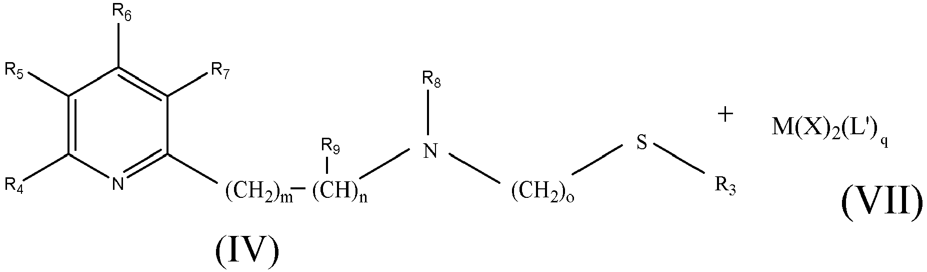 wherein q is 1, 2 or 3 and
all other substituents have the meanings as defined above.

The process to obtain the catalyst (RS2) is usually carried out in a solvent (or a mixture of solvents). Suitable solvents are esters, ethers, amides, hydrocarbons, and alcohols. Preferred solvents are toluene, ethyl acetate, THF and diglyme.

The process to obtain the catalyst is usually carried out at elevated temperature (50 - 180°).

The process to obtain the catalyst is usually carried out at ambient pressure

The obtained catalyst (in crystalline form) are filtered off and they can be further purified.

As stated above the obtained catalysts are used in the selective reductions (selective hydrogenations), wherein the yield and selectivity of the desired product is excellent.

### Reduction process

The reduction process (selective hydrogenation) of the compound of formula (I) can be carried out according to the following reaction scheme 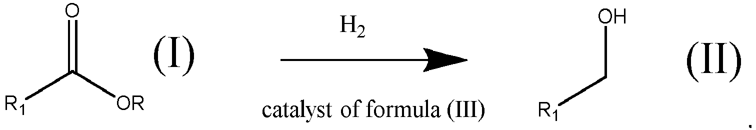 wherein all substituents have meanings as defined above.

In these hydrogenation processes H₂ is added in form of a gas (pure H₂ gas or part or a mixture).

The catalyst of formula (III) is usually used in an amount of 0.001 - 0.5 mol-% (based on the number of moles of the compounds of formula (I)).

Therefore the present invention relates to a process (P4), which is process (P), (P1), (P1'), (P1"), (P1'''), (P1""), (P2), (P2'), (P2"), (P3), (P3') or (P3"), wherein the at least one catalyst of formula (III) is used in an amount of 0.001 - 0.5 mol-% (based on the number of moles of the compounds of formula (I)).

The hydrogenation process can be carried out with (pure) H₂ gas or with a gas which comprises H₂. Preferably the hydrogenation process according to the present invention is carried out with (pure) H₂ gas.

Therefore the present invention relates to a process (P5), which is process (P), (P1), (P1'), (P1"), (P1'''), (P1""), (P2), (P2'), (P2"), (P3), (P3'), (P3") or (P4), wherein the hydrogenation is carried out with (pure) H₂ gas or with a gas which comprises H₂. Preferably the hydrogenation process according to the present invention is carried out with (pure) H₂ gas.

Therefore the present invention relates to a process (P5'), which is process (P), (P1), (P1'), (P1"), (P1'''), (P1""), (P2), (P2'), (P2"), (P3), (P3'), (P3") or (P4), wherein the hydrogenation is carried out with pure H₂ gas.

The hydrogenation process can be carried out at ambient pressure as well as at elevated pressure. Preferably the hydrogenation process according to the present invention is carried out at elevated pressure (10 - 50bar), usually in an autoclave (or any other vessel, which can resist the pressure.

Therefore the present invention relates to a process (P6), which is process (P), (P1), (P1'), (P1"), (P1'''), (P1""), (P2), (P2'), (P2"), (P3), (P3'), (P3"), (P4), (P5) or (P5'), wherein the hydrogenation is carried out at ambient pressure.

Therefore the present invention relates to a process (P6'), which is process (P), (P1), (P1'), (P1"), (P1'''), (P1""), (P2), (P2'), (P2"), (P3), (P3'), (P3"), (P4), (P5) or (P5'), wherein the hydrogenation is carried out at elevated pressure (10 - 50bar).

The hydrogenation can be carried out in a solvent (or mixture of solvents). Suitable solvents are esters, ethers, amides, hydrocarbons, halogenated hydrocarbons and alcohols. Preferred solvents are CH₂Cl₂, toluene, ethyl acetate, THF, methanol, ethanol and isopropanol, especially preferred solvents are toluene and isopropanol.

Therefore the present invention relates to a process (P7), which is process (P), (P1), (P1'), (P1"), (P1'''), (P1""), (P2), (P2'), (P2"), (P3), (P3'), (P3"), (P4), (P5), (P5'), (P6) or (P6'), wherein the hydrogenation is carried out carried out in at least one solvent.

Therefore the present invention relates to a process (P7'), which is process (P), (P1), (P1'), (P1"), (P1'''), (P1""), (P2), (P2'), (P2"), (P3), (P3'), (P3"), (P4), (P5), (P5'), (P6) or (P6'), wherein the hydrogenation is carried out carried out in at least one solvent chosen from the group consisting of esters, ethers, amides, hydrocarbons, halogenated hydrocarbons and alcohols.

Therefore the present invention relates to a process (P7"), which is process (P), (P1), (P1'), (P1"), (P1'''), (P1""), (P2), (P2'), (P2"), (P3), (P3'), (P3"), (P4), (P5), (P5'), (P6) or (P6'), wherein the hydrogenation is carried out carried out in at least one solvent chosen from the group consisting of CH₂Cl₂, toluene, ethyl acetate, THF, methanol, ethanol and isopropanol (especially preferred are toluene and isopropanol).

The hydrogenation is usually carried out at an elevated temperature (30 - 150 °C).

Therefore the present invention relates to a process (P8), which is process (P), (P1), (P1'), (P1"), (P1'''), (P1""), (P2), (P2'), (P2"), (P3), (P3'), (P3"), (P4), (P5), (P5'), (P6), (P6'), (P7), (P7') or (P7"), wherein the hydrogenation is carried out carried out at an elevated temperature (30 - 150 °C).

It is also possible to reduce the compound of formula (I) selectively by a transfer hydrogenation process. In that case no H₂ gas needs to be added. As reductant any suitable hydrogen donor can be used, including secondary alcohols, such as isopropanol and formic acid, its salts or derivatives.

Therefore the present invention relates to a process (P9), which is process (P), (P1), (P1'), (P1"), (P1'''), (P1""), (P2), (P2'), (P2"), (P3), (P3'), (P3") or (P4), wherein the hydrogenation is a transfer hydrogenation.

The following examples serve to illustrate the invention. If not otherwise stated the temperature is given in °C.

### Examples

### General:

Transition metal precursors, reagent and solvents were obtained from commercial sources and used as received unless noted otherwise. GC analysis was carried out on an Agilent 7890B GC system with a HP-5 normal-phase silica column, using Helium as a carrier gas and dodecane as an internal standard. NMR spectra were recorded on a Bruker AV400, Bruker AV300 or Bruker Fourier300 NMR spectrometer. ¹H and ¹³C-NMR spectra were referenced w.r.t. the solvent signal. Chemical shifts are in ppm, coupling constants in Hz. HR-MS measurements were recorded on an Agilent 6210 Time-of-Flight LC/MS, peaks as listed correspond to the highest abundant peak and are of the expected isotope pattern.

### Ligand synthesis

### Example 1: 2-(ethylthio)-N-((6-methylpyridin-2-yl)methyl)ethan-1-amine [ligand of formula (IVg)]

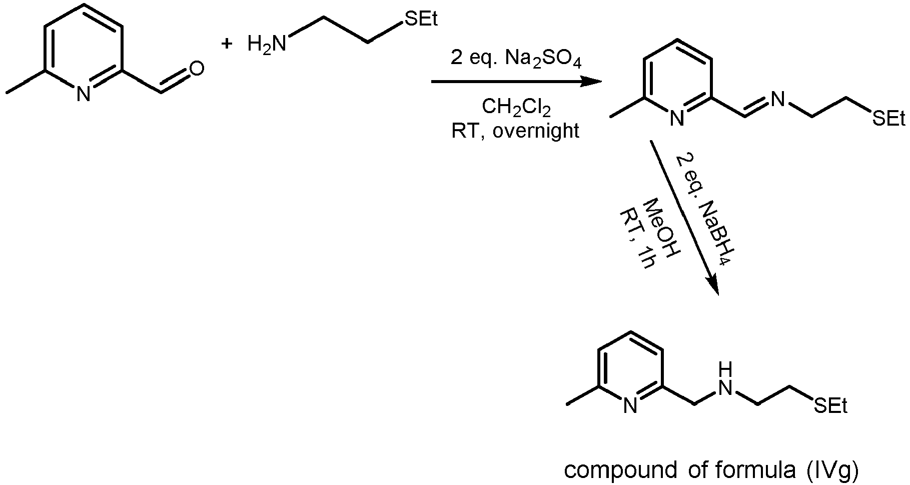

6-methylpyridine-2-carboxaldehyde (3.0 g, 25 mmol) and 2-(Ethylthio)ethylamine (2.63 g, 2.8 mL, 25 mmol) were dissolved in CH₂Cl₂ (75 mL), then Na₂SO₄ (7.1 g, 50 mmol) was added. The suspension was stirred at room temperature overnight, filtered and the filter cake was washed with CH₂Cl₂. The combined volatiles were removed *in vacuo,* yielding 5.45 g of imine as brown oil, which was used directly in the following step without further purification. Therefore, the imine was dissolved in MeOH (50 mL) and NaBH₄ (1.9 g, 51 mmol) was added portionwise at 0 °C. The mixture was stirred at room temperature for another hour, after which the solvent was removed *in vacuo.* Then CH₂Cl₂ (20 mL) and water (20 mL) were added. The aqueous layer was extracted with CH₂Cl₂ (three times 20 mL). The combined organic layers were washed with brine (20 mL) and dried over Na₂SO₄. Evaporating the solvent and drying *in vacuo* yielded 4.95 g (94%) of the ligand of formula (IVg) as an orange oil, which was directly used for complex synthesis.

¹H-NMR (300 MHz, CDCl₃): δ 7.45 (t, 1H, *J* = 7.6, CHₐᵣₒₘ), 7.07 (d, 1H, *J* = 7.8, CHₐᵣₒₘ), 6.96 (d, 1H, *J* = 7.5, CHₐᵣₒₘ), 3.84 (s, 2H), 2.80 (dt, 2H), 2.66 (dt, 2H), 2.48 (m, 5H), 1.23 (t, 3H, *J* = 7.4) ppm.

¹³C-NMR (75 MHz, CDCl₃): δ 158.9, 157.8, 136.5, 121.3, 118.9, 54.9, 48.2, 31.8, 25.6, 24.4 ppm.

HRMS (ESI+): calculated for C₁₁H₁₈N₂S: 210.1191; found 211.1265 (M+H), 233.1082 (M+Na).

### Example 2: 2-(methylthio)-N-((pyridin-2-yl)methyl)ethan-1-amine [ligand of formula (IVa)]

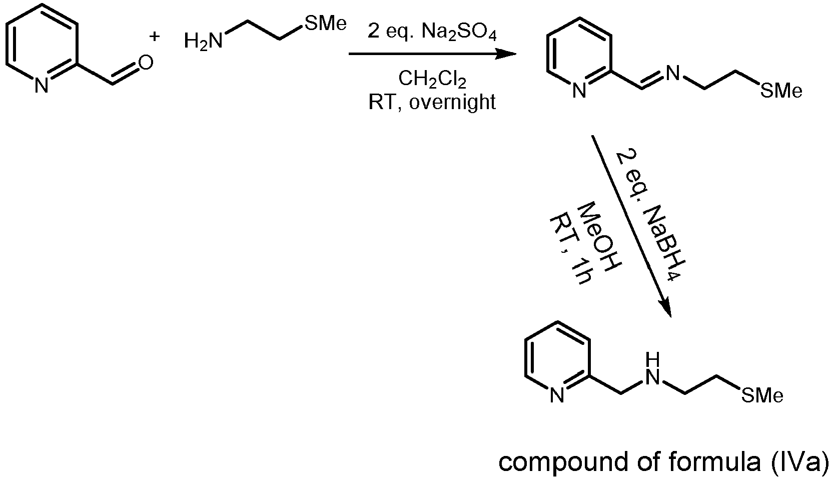

The ligand of formula (IVa) was prepared in analogy to Example 1.

¹H NMR (300 MHz, CD₂Cl₂) δ 8.43 (ddd, 1H, *J* = 4.9 Hz, *J* = 1.8 Hz, *J* = 0.9 Hz, CHₐᵣₒₘ), 7.57 (td, 1H, *J* = 7.7 Hz, *J* = 1.8 Hz, CHₐᵣₒₘ), 7.24 (d, 1H, *J* = 7.8 Hz, CHₐᵣₒₘ), 7.07 (dd, 1H, *J* = 7.5 Hz, *J* = 5.0.7 Hz, CHₐᵣₒₘ), 3.81 (s, 2H), 2.75 (td, 2H, *J* = 6.5 Hz, *J* = 0.8 Hz, CH₂), 2.58 (td, 2H, *J* = 6.5 Hz, *J* = 0.6 Hz, CH₂), 1.99 (s, 3H, CH₃) ppm.

¹³C NMR (75 MHz, CD₂Cl₂): δ 160.2, 149.1, 136.2, 121.9, 121.7, 54.8, 47.6, 34.4, 15.0 ppm.

HRMS (ESI+): calculated for C₉H₁₄N₂S: 182.0878 (M+H): 183.0950; found 183.0950 (M+H).

### Example 3: 2-(ethylthio)-N-((pyridin-2-yl)methyl)ethan-1-amine [ligand of formula (IVb)]

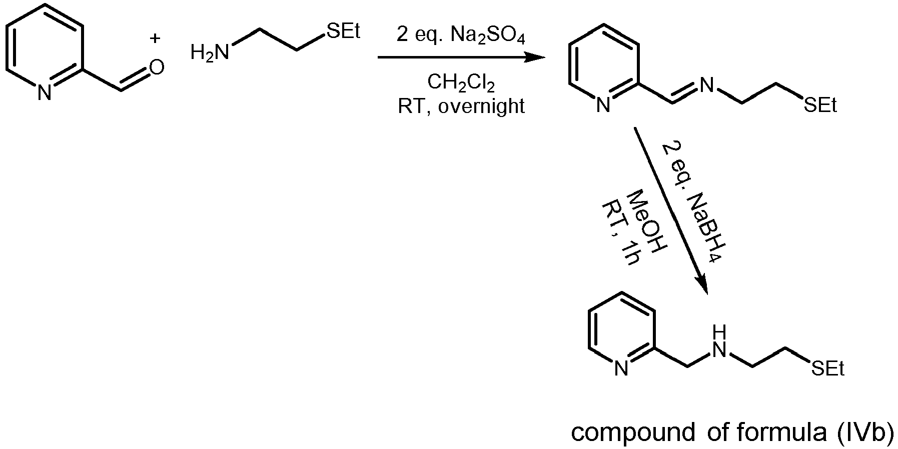

The ligand of formula (IVb) was prepared according to Example 1.

¹H NMR (300 MHz, CD₂Cl₂): δ 8.51 (ddd, 1H, *J* = 4,8 Hz, *J* = 1.5 Hz, *J* = 0.9 Hz, CHₐᵣₒₘ), 7.64 (td, 1H, *J* = 7.5 Hz, *J* = 1.8 Hz, CHₐᵣₒₘ), 7.32 (d, 1H, *J* = 7,8 Hz, CHₐᵣₒₘ), 7.19 -7.12 (m, 1H, CHₐᵣₒₘ), 3.88 (s, 2H, CH₂), 2.85 - 2.79 (m, 2H, CH₂), 2.72-2.66 (m, 2H, CH₂), 2.52 (q, 2H, *J* = 7.5 Hz, CH₂), 2.09 (d, 1H, *J* = 9.6 Hz, NH), 1.23 (t, 3H, *J* = 7.4 Hz, CH₃) ppm.

¹³C NMR (75 MHz, CD₂Cl₂): δ 161.6, 149.7, 136.8, 122.5, 122.3, 55.4, 48.9, 32.5, 26.2, 15.3 ppm.

HRMS (ESI+): calculated for C₁₀H₁₆N₂S: 196.1034; (M+H): 197.1107; (M+Na): 219.0926; found 197.1108 (M+H), 219.0929 (M+Na).

### Example 4: 2-(ethylthio)-N-((6-methoxy-pyridin-2-yl)methyl)ethan-1-amine [ligand of formula (IVk)]

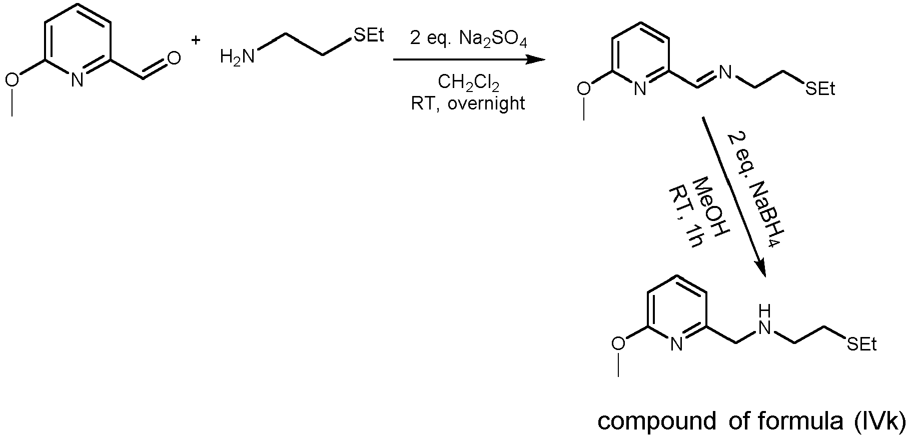

The ligand of formula (IVk) was prepared according to Example 1 in a 84% yield. ¹H-NMR (300 MHz, CDCl₃): δ 7.54 (dd, 1H, *J* = 8.1, *J* = 7.4, CHₐᵣₒₘ), 6.87 (d, 1H, *J* = 7,2), 6.63 (d, 1H, *J* = 8.1), 4.55 (s, NH), 3.92 (s, 3H), 3.90 (m, NH), 3.80 (s, 2H), 2.83 (t, 2H, *J* = 6.5), 2.66 (t, 2H, *J* = 6.5), 2.52 (t, 2H, *J* = 7.5), 1.23 (t, 3H, *J* = 7.2) ppm. ¹³C-NMR (75 MHz, CDCl₃): δ 163.8,157.3,138.8,114.5,108.7, 54.3, 53.2,48.1, 32.0, 25.8, 14.8 ppm.

HRMS (ESI+): calculated for C₁₁H₁₈N₂OS: 227.1213 (M+H); found 227.1217 (M+H).

### Example 5: 2-(ethylthio)-N-((quinolin-2-yl)methyl)ethan-1-amine [ligand of formula (IVI)]

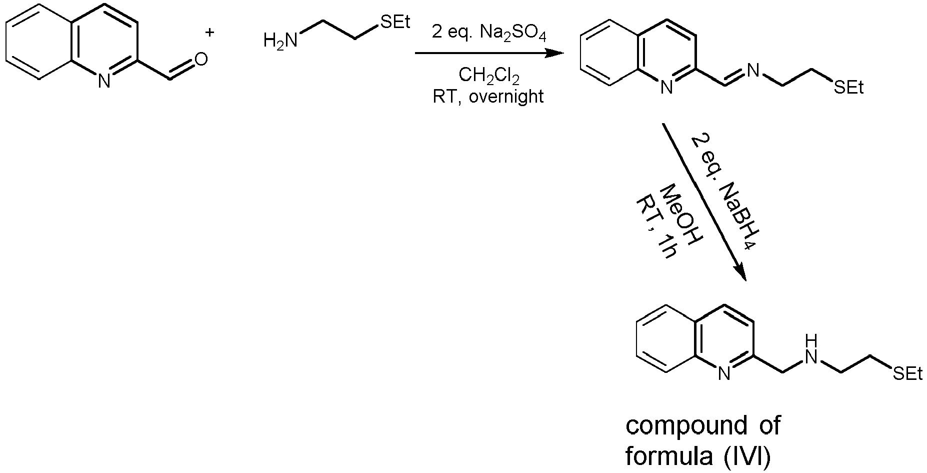

The ligand of formula (IVI) was prepared according to Example 1 and purification by Kugelrohr distillation.

¹H NMR (300 MHz, CD₂Cl₂): δ 8.13 (d, 1H, *J* = 8.4 Hz, CHₐᵣₒₘ), 8.00 (d, 1H, *J* = 8.7 Hz, CHₐᵣₒₘ), 7.82 (dd, 1H, *J* = 8.3 Hz, *J* = 1.5 Hz, CHₐᵣₒₘ), 7.69 (ddd, 3H, *J* = 8.5 Hz, *J* = 6.9 Hz, *J* = 1.5 Hz, CHₐᵣₒₘ), 7.55 - 7.45 (m, 2H, CHₐᵣₒₘ), 4.08 (s, 2H, CH₂), 2.89 (td, 2H, *J* = 6.8 Hz, *J* = 1.2 Hz, CH₂), 2.73 (td, 2H, *J* = 6.4 Hz, *J* = 0.9 Hz, CH₂), 2.55 (q, 2H, *J* = 7.4 Hz, CH₂), 2.14 (d, 1H, *J* = 11.4 Hz, NH), 1.24 (t, 3H, *J* = 7.4 Hz, CH₃) ppm.

¹³C NMR (75 MHz, CD₂Cl₂): δ 161.5, 136.7, 129.8, 129.5, 128.1, 127.9, 126.5, 121.0, 56.0, 49.1, 32.6, 26.2, 15.29 ppm.

HRMS (ESI+): calculated for C₁₄H₁₈N₂S: 246.1191; (M+H): 247.1264; found 247.1267 (M+H)

### Example 6: 2-(ethylthio)-N-(1-(pyridin-2-yl)ethyl)ethan-1-amine [ligand or formula (IVe)]

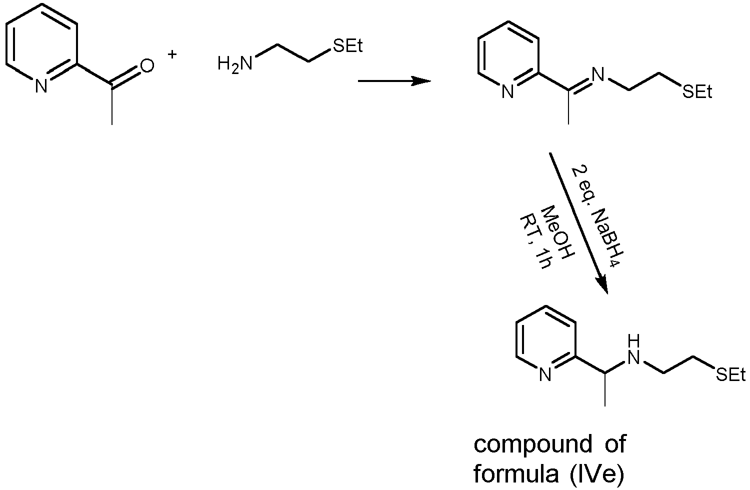

The ligand of formula (IVe) was prepared according to Example 1 with imine formation performed in the presence of 5 mol% of p-toluenesulfonic acid in toluene under reflux conditions and purification by Kugelrohr distillation.

¹H NMR (300 MHz, CD₂Cl₂): δ 8.51 (ddd, 1H, *J* = 4.8 Hz, *J* = 1.9 Hz, *J* = 1.0 Hz, CHₐᵣₒₘ), 7.64 (td, 1H, *J* = 7.6 Hz, *J* = 1.8 Hz, CHₐᵣₒₘ), 7.32 (dt, 1H, *J* = 7.8 Hz, *J* = 1.1 Hz, CHₐᵣₒₘ), 7.14 (ddt, 1H, *J* = 7.5 Hz, *J* = 4.8 Hz, *J* = 1.2 Hz, CHₐᵣₒₘ), 3.84 (q, 1H, *J* = 6.9 Hz, CH), 2.71 - 2.55 (m, 4H, CH₂), 2.47 (q, 2H, *J* = 7.4 Hz, CH₂), 2.05 (d, 1H, *J* = 39.3 Hz, NH), 1.34 (d, 3H, *J* = 6.9 Hz, CH₃), 1.20 (d, 3H, *J* = 7.5 Hz, CH₃) ppm. ¹³C NMR (75 MHz, CD₂Cl₂): δ 165.4, 149.7, 136.9, 122.3, 121.4, 59.7, 47.1, 32.7, 26.1, 23.2, 15.2 ppm.

HRMS (ESI+): calculated for C₁₁H₁₈N₂S: 210.1191; (M+H), 211.1264; (M+Na): 233.1083; found 211.1265 (M+H), 233.1083 (M+Na).

### Example 7: 2-(ethylthio)-N-methyl-N-(pyridin-2-ylmethyl)ethan-1-amine [ligand of formula (IVd)]

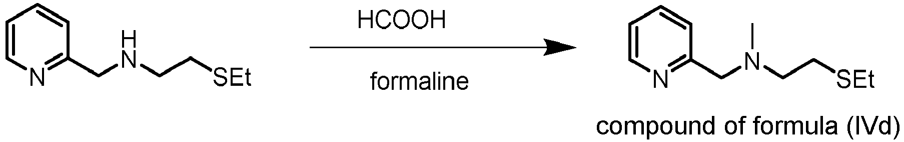

2-(Ethylthio)-N-(pyridin-2-ylmethyl)ethan-1-amine (ligand of formula (IVb), 850 mg, 3.75 mmol), formalin (4 mL of 37% wt formaldehyde in water) and formic acid (4 mL) were stirred at 70°C overnight. All volatiles were removed *in vacuo* and CH₂Cl₂ (10 mL) and saturated NaHCO₃ solution (10 mL) were added. The aqueous layer was extracted with CH₂Cl₂ (three times 10 mL). The combined organic layers were washed with brine (20 mL) and dried over Na₂SO₄. Removal of the solvent yielded 754 mg (3.59 mmol, 96%) of 2-(ethylthio)-*N*-methyl-*N*-(pyridin-2-ylmethyl)ethan-1-amine as an orange liquid (p = 1.081 g cm⁻³). The ligand of formula (IVb) was further purified by Kugelrohr distillation.

¹H-NMR (300 MHz, CDCl₃): δ 8.46 (d, 1H, *J* = 5.1, CHₐᵣₒₘ), 7.58 (dt, 1H, *J* = 7.8, *J* = 1.8, CHₐᵣₒₘ), 7.38 (d, 1H, *J* = 7.8, CHₐᵣₒₘ), 7.08 (ddd, 1H, *J* = 7.5, *J* = 4.8, *J* = 1.2, CHₐᵣₒₘ), 3.62 (s, 2H), 2.62 (s, 4H), 2.45 (q, 2H, *J* = 7.4), 2.31 (s, 3H, N-CH3), 1.17 (t, 3H, *J* = 7.4) ppm.

¹³C NMR (101 MHz, CDCl₃): δ 159.2, 149.0, 136.4, 123.1, 122.0, 63.6, 57.3, 56.9, 42.4, 31.9, 29.3, 26.1, 14.8 ppm.

HRMS (ESI+): calculated for C₁₁H₁₈N₂S: 210.1191; found 211.1265 (M+H), 233.1084 (M+Na)

### Catalyst synthesis

### Example 8: Ru(6-MeNNS^{Et})(PPh₃)Cl₂

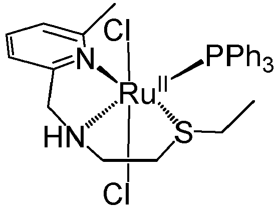

RuCl₂(PPh₃)₃ (1g, 1.04 mmol) and the ligand of formula (IVg) (obtained from Example 1) (231.4 mg, 1.1 mmol) were placed in a 25 mL Schlenk tube under argon atmosphere, and dissolved in dry diglyme (2 mL). The reaction mixture was heated to 165°C for 2 h, allowed to cool down to room temperature and stored at -18°C to precipitate further overnight. Cold Et₂O (2 mL) was added while cooling with a dry ice/*iso*-propanol bath. The precipitate was filtrated by cannula, and washed with Et₂O (5 times 2 mL). The orange powder was dried *in vacuo,* affording 530 mg (79%) of Ru(6-MeNN-S^{Et})(PPh₃)Cl₂ as an orange powder. An equilibrium of two conformations of Ru(6-MeNNS^{Et})(pPh₃)Cl₂ are existent in solution, delivering a doubled set of signals in NMR. For ¹H-NMR only data of the major conformation is given due to overlapping signals.

¹H-NMR (300 MHz, CD₂Cl₂): δ 7.67-7.16 (m, 17H, CHₐᵣₒₘ), 7.01 (d, 1H, *J* = 7.8, CHₐᵣₒₘ), 5.65 (m, 2H), 4.47 (m, 1H), 3.5 (m, 1H), 3.34 (m, 1H), 3.22 (d, 1H, *J* = 11.1), 2.98 (m, 1H), 2.59 (m, 1H), 1.53 (m, 2H), 0.87 (t, 3H, *J* = 7.5) ppm.

³¹P-NMR (122 MHz, CD₂Cl₂): δ 48.8, 45.8 ppm.

HRMS (ESI+): calculated for C₂₉H₃₂Cl₂N₂PRuS (M+H): 644.0518; found 644.0518 (M+H), 667.0412 (M+Na)

### Example 9: Ru(NNs^{Me})(PPh₃)Cl₂

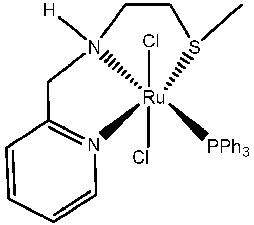

*Ru(NNS^{Me})(PPh₃)Cl₂* was prepared according to Example 8. An equilibrium of two conformations was obtained.

¹H-NMR (300 MHz, CD₂Cl₂): δ 8.47 (d, 1H, *J* = 5.7), 7.72 (m, 1H), 7.56 (m, 6H), 7.32 (m, 10H), 6.86 (t, 1H, *J* = 6.3), 5.45 (s, broad, 1H, NH), 5.20 (t, 1H, *J* = 12.6), 4.38 (m, 1H), 3.41 (m, 2H), 3.26 (d, 1H, *J* = 11.1), 2.55 (m, 1H), 1.50 (s, 3H).

³¹P-NMR (122 MHz, CD₂Cl₂): δ 51.8, 50.7

HRMS (ESI+): calculated for C₂₇H₂₉Cl₂N₂PRuS: 616.0210 (M+); found 616.0197 (M+)

### Example 10: Ru(NNS^{Et})(PPh₃)Cl₂

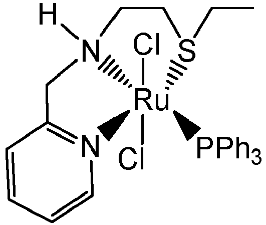

Ru(NNS^{Et})(PPh₃)Cl₂ was prepared according to Example 8. An equilibrium of two conformations was obtained in 84% yield.

¹H-NMR (300 MHz, CD₂Cl₂): δ 8.45 (d, 1H, *J* = 5.7), 7.72 (m, 1H), 7.57 (m, 6H), 7.34 (m, 10H), 6.86 (t, 1H, *J* = 6.3), 5.49 (s, broad, 1H, NH), 5.22 (t, 1H, *J* = 13.5), 4.40 (m, 1H), 3.47 (m, 2H), 3.36 (m, 1H), 2.80 (m, 1H), 2.52 (m, 1H), 1.27 (m, 2H), 1.19 (m, 1H), 0.95 (t, 3H, *J* = 7.5)

³¹P-NMR (122 MHz, CD₂Cl₂): δ 51.8, 50.7

HRMS (ESI+): calculated for C₂₈H₃₁Cl₂N₂PRuS: 630.0366 (M+); found 630.0388 (M+), 653.0270 (M+Na)

### Example 11: Ru(6-MeONNS^{Et})(PPh₃)Cl₂

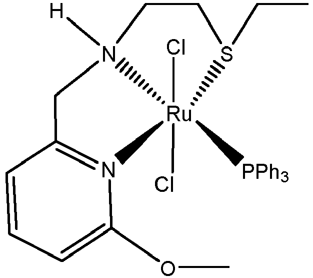

Ru(6-MeONNS^{Et})(PPh₃)Cl₂ was prepared according to Example 8. An equilibrium of two conformations was obtained in 88% yield.

¹H-NMR (400 MHz, CD₂Cl₂): δ 7.94 (m, 2H), 7.65 (m, 2H), 7.42-7.14 (m, 12H), 7.07 (d, 1H, *J* = 7.6), 6.56 (d, 1H, *J* = 8.4), 5.56-5.36 (m, 2H), 4.46 (m, 1H), 3.50-3.19 (m ,2H), 3.21 (dd, 1H, *J* = 11.0, *J* = 2.2), 2. 87 (m, 1H), 2.83 (s, 3H, twinned), 2.50 (m, 1H), 1.33 (m, 1H), 0.87 (t, 3H, twinned, overlapping)

³¹P-NMR (122 MHz, CD₂Cl₂): δ 47.2, 45.9

HRMS (ESI+): calculated for C₂₉H₃₂Cl₂N₂OPRuS (M+H): 660.0468; found: 660.0469 (M+H), 683.0363 (M+Na)

### Example 12: Ru(QuinNS^{Et})(PPh₃)Cl₂

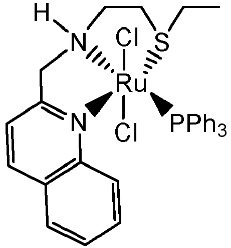

Ru(QuinNS^{Et})(PPh₃)Cl₂ was prepared according to Example 8. An equilibrium of two conformations was obtained.

¹H-NMR (300 MHz, CD₂Cl₂): δ 8.12 (d, 2H, *J* = 8.4), 7.74-6.66 (m, 19H), 5.90 (s, broad, NH), 5.74 (t, 1H, *J* = 13.3), 4.72 (m, 1H), 3.58-3.40 (m, 3H), 3.05 (m, 1H), 2.72 (m, 1H), 1.66 (m, 1H), 0.95 (t, 3H, *J* = 7.5)

³¹P NMR (122 MHz, CD₂Cl₂): δ 48.90, 45.86

HRMS (ESI+): calcd. for C₃₂H₃₃Cl₂N₂PRuS: 680.0519 (M+); found 680.0500 (M+)

### Example 13: Ru(N-Me-NS^{Et})(PPh₃)Cl₂

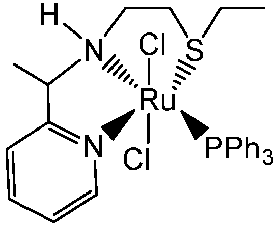

Ru(N-Me-NS^{Et})(PPh₃)Cl₂ was prepared according to Example 8. An equilibrium of two conformations was obtained.

¹H-NMR (300 MHz, CD₂Cl₂): δ 8.53 (d, 1H, *J* = 5.7), 7.72 (m, 1H), 7.57 (m, 6H), 7.33 (m, 10H), 6.85 (t, 1H, *J* = 6.6), 5.35 (m, 1H), 4.93 (s, broad, NH), 3.68-3.31 (m, 3H), 2.81 (m, 1H), 2.53 (m, 1H), 1.80 (d, 3H, *J* = 6.9), 1.25 (m, 1H), 0.97 (t, 3H, *J* = 7.2) ³¹P NMR (122 MHz, CD₂Cl₂): δ 51.5, 50.3

HRMS (ESI+): calculated for C₂₉H₃₃Cl₂N₂PRuS: 644.0518 (M+); found 644.0513 (M+)

### Example 14: Ru(NN^{Me}S^{Et})(PPh₃)Cl₂

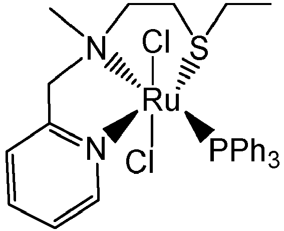

Ru(NN^{Me}S^{Et})(PPh₃)Cl₂ was prepared according to Example 8 . An equilibrium of two conformations was obtained in 54%.

¹H-NMR (300 MHz, CD₂Cl₂): δ 8.11 (d, 1H, *J =* 5.7), 7.92 (m, 6H), 7.47 (dt, 1H, *J* = 7.5, *J =* 1.5), 7.30 (m, 10H), 6.56 (t, 1H, *J* = 7.5), 5.67 (d, 1H, *J* = 14.4), 3.87 (d, 1H, *J* = 14.4), 3.15 (s, 3H), 2.86 (m, 1H), 2.70 (m, 1H), 2.30 (m, 2H), 0.74 (m, 1H), 0.67 (t, 3H, *J* = 6.9), 0.42 (m, 1H)

³¹P-NMR (122 MHz, CD₂Cl₂): δ 51.4, 50.4

HRMS (ESI+): calculated for C₂₉H₃₃Cl₂N₂PRuS: 644.0518 (M+); found 644.0505 (M+)

### HYDROGENATION REACTIONS

### Example 15: selective hydrogenation of a specific ester

The compounds of formulae (A) were hydrogenated.

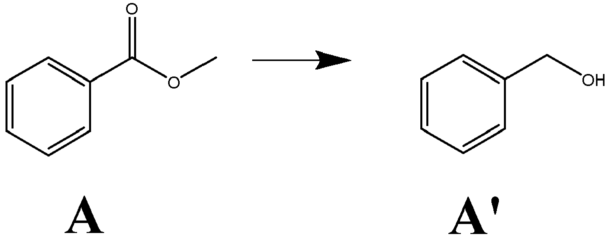

4 mL glass reaction vials and stirring bars were dried overnight at 110°C, closed with PTFE/rubber septa, placed in a multiple reactor inlet suitable for a pressure vessel, and brought under argon atmosphere by three vacuum-argon cycles. VVith a syringe the reaction vessels were charged with the catalyst as stock solution in *i*PrOH (1 mL, 0.0005 mol/L, 0.05 mol%), followed by a solution of the compound A,in *i*PrOH (1 mL, 1 mol/L, 1 mmol). After that a solution of freshly sublimed base in THF (12.5 µL, 1 mol/L, 0.0125 mmol, 1.25 mol%) was added with a syringe. The reaction mixtures were transferred to an argon-filled pressure vessel, which was immediately flushed with three nitrogen and three hydrogen cycles, then pressurized to 30 bar hydrogen, heated to 80°C and stirred for 16h. After that the pressure vessel was cooled down to room temperature and depressurized. The reaction mixtures were filtered over silica and rinsed with ethanol (2 mL). The products are determined based on GC analysis retention time. The given values [%] are related to GC area%.

The results are summarized in the following table.

**Table 1: hydrogenation of the compound of formula (A)**

| **Exp.** | **Cat.** | **Base** | **Conversion** | **Product Compund A'** | |
|---|---|---|---|---|---|
| | **0.05 mol%** | **1-2 mol%** | **C [%]** | **Y [%]** | **S [%]** |
| **15a** | Cat of Exp.9 | KO*t*Bu | 100 | 100 | 100 |
| **15b** | Cat of Exp.10 | KO*t*Bu | 100 | 99 | 99 |
| **15c** | Cat of Exp.10 | LiO*t*Bu | 100 | 99 | 99 |

### Example 16

In a similar manner as described in Example 15 methyl hexanoate was hydrogenated to 1-hexanol. In this experiment the catalyst of Exp 9 was used and NaOtBu was used as base. The ratios between substrate base and catalyst were 262 : 29 : 1. The temperature was 100°C and the hydrogen pressure 30 bar. After 16h the solution was analysed and 1-hexanol was found in 43% yield.

### Example 17

A 100 mL hastelloy autoclave with mechanical stirrer was charged with the catalyst of example 9 (3.3 mg, 0.005 mmol), methyl stearate (2,98 g, 10 mmol), 20 mL of toluene, and freshly sublimed KOtBu (7 mg, 0.0625 mmol, 1.25 mol%) under an argon atmosphere. The autoclave vessel was then pressurized to 30 bar hydrogen, heated to 100°C and stirred for 16 hours. The pressure vessel was cooled down to room temperature and depressurized. Removal of the solvent in vacuo yielded 2.7 g of stearyl alcohol as an off-white flaky powder.

1H NMR (300 MHz, CDCl3): δ 3.69 (dt, 2H, J = 6.6; J = 5.4 Hz), 1.62 (m, 2H), 1.30 (m, 31 H), 0.93 (t, 3H, J = 6.3 Hz) ppm.

GC-MS (ESI-): single component, calculated for C18H380: 270 (M); found 269 (M-H).

### Example 18: Hydrogenation of cinnamate esters

A 100 mL hastelloy autoclave with mechanical stirrer was charged with the catalyst of example 9 (23 mg, 0.038 mmol, 0.25 mol%), substrate (15 mmol), 30 mL of toluene, freshly sublimed KOtBu (41 mg, 0.38 mmol, 2.5 mol%), and 1000 µl of anhydrous n-dodecane under an argon atmosphere. The autoclave vessel was flushed with nitrogen three times, and with hydrogen two times, then pressurized to 30 bar H2, heated to 40°C and stirred for 4 hours. During the reaction time the pressure was kept at 30 bar H₂. The products are determined based on GC analysis retention time. The given values for conversion (C), yield (Y), and selectivity (S) [%] are mol% with regard to the initial cinnamyl ester amount, and corrected by n-dodecane. The results are summarized in the following table.

**Table 2: hydrogenation of cinnamate esters**

| **Exp** | **Substrate** | **T [°C]** | **t [h]** | **C [%]** | **S [%]** | **Y [%]** |
|---|---|---|---|---|---|---|
| **18a** | Methyl cinnamate | 40 | 4 | >99 | 90 | 90 |
| **18b** | Isobutyl cinnamate | 40 | 4 | >99 | 95 | 95 |

### Example 19: Hydrogenation of 5-methyldihydrofuran-2(3H)-one

The catalyst of example 9 (23 mg, 0.038 mmol, 0.25 mol%), 5-methyldihydrofuran-2(3H)-one (1.46 g, 15 mmol), 30 mL of toluene, and freshly sublimed KOtBu (41 mg, 0.38 mmol, 2.5 mol%) were reacted according to the method in Example 18. An amount of 25 mL of the reaction mixture was used for the product purification. Column chromatography yielded 1.399 g (92%) of pentane-1,4-diol.

### Example 20: Hydrogenation of methyl cyclohex-1-ene-1-carboxylate

Methyl cyclohex-1-ene-1-carboxylate was hydrogenated according to Example 18. The reaction mixture was initially heated to 60°C. After stirring for 1 hour the vessel was allowed to cool down to 40°C and was kept at this temperature under stirring for 5 hours. Column chromatography yielded 0.75 g (63%) of cyclohex-1-en-1-ylmethanol.

## Claims

1. A process of production of a compound of formula (II) 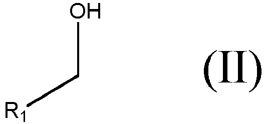 wherein
R₁ is an aromatic ring system which is unsubstituted or an aromatic ring system which is substituted; a heteroaromatic ring system which is unsubstituted or a heteroaromatic ring system which is substituted; an aliphatic ring system which is unsubstituted or an aliphatic ring system which is substituted; -CH₃;-CH₂CH₃; an unsubstituted C₃ - C₂₂ alkyl group, which can be linear or branched and which can also be partially unsaturated; or an substituted C₂ - C₂₂ alkyl group, which can be linear or branched and which can also be partially unsaturated
by a selective reduction of a compound of formula (I) 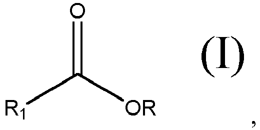 wherein
R is a linear C₁-C₆-alkyl group, which can be substituted; a branched C₃-C₆-alkyl group, which can be substituted or a benzyl group, which can be substituted, and
R₁ is an aromatic ring system which is unsubstituted or an aromatic ring system which is substituted; a heteroaromatic ring system which is unsubstituted or a heteroaromatic ring system which is substituted; an aliphatic ring system which is unsubstituted or an aliphatic ring system which is substituted; -CH₃;-CH₂CH₃; an unsubstituted C₃ - C₂₂ alkyl group, which can be linear or branched and which can also be partially unsaturated ; or an substituted C₂ - C₂₂ alkyl group, which can be linear or branched and which can also be partially unsaturated,
or R and R₁ form together a 4 to 7 membered ring system, which can be substituted,
**characterised in that** the selective reduction is carried out in the presence of at least one transition metal catalyst of formula (III)
[M(L)(X)ₐ(L')_{b}] **(III)**,
wherein
M is a transition metal and
X is an anion, and
L' is a monodentate ligand, and
L is a tridentate ligand of formula (IV) 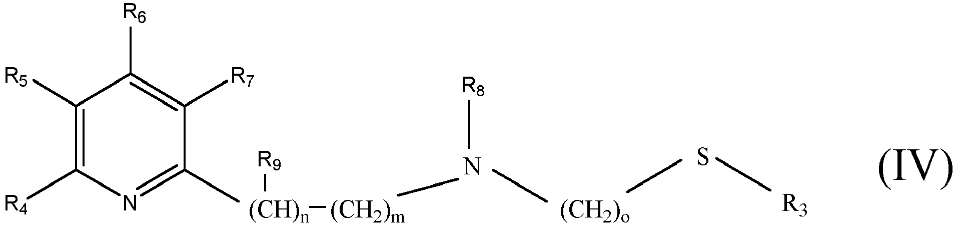 wherein
R₃ is a linear C₁ - C₄ alkyl group, which can be substituted; a branched C₃ - C₄ alkyl group, which can be substituted; or a phenyl group, which can be substituted, and
R₄ is H; a linear C₁ - C₄ alkyl group, which can be substituted; a branched C₃ - C₄ alkyl group, which can be substituted; or OC₁-C₂alkyl, and
R₅ is H; a linear C₁ - C₄ alkyl group, which can be substituted; a branched C₃ - C₄ alkyl group, which can be substituted; or OC₁-C₂alkyl,
or R₄ and R₅ form a C₄-C₈ ring system, which can be aliphatic or aromatic, and
R₆ is H; a linear C₁ - C₄ alkyl group, which can be substituted; a branched C₃ - C₄ alkyl group, which can be substituted;or OC₁-C₂alkyl, and
R₇ is H; a linear C₁ - C₄ alkyl group, which can be substituted; a branched C₃ - C₄ alkyl group, which can be substituted;or OC₁-C₂alkyl, and
R₈ is H or a linear C₁ - C₄ alkyl group, which can be substituted; a branched C₃ - C₄ alkyl group, which can be substituted, and
R₉ is -CH₃ or -CH₂CH₃, and
m is 0, 1 or 2, and
n is 0, 1 or 2,
with the proviso that the sum of m+n is 1 or 2,
o is 2 or 3,
a is 0, 1, 2, or 3,
b is 0, 1, 2, or 3,
with the proviso that the sum of a+b is 2, 3 or 4.

2. Process according to claim 1, wherein the process is carried out in the presence of at least one base.

3. Process according to claim 1 or 2, wherein the process is carried out in the presence of at least one base of formula (VIII)
M¹(OC₁-C₅alkyl) (VIII),
wherein M¹ is an alkali metal.

4. Process according to claim 1 or 2, wherein the process is carried out in the presence of at least one base of formula (VIII'),
M¹(OC₃-C₅alkyl) (VIII')
wherein
M¹ is Li, Na or K.

5. Process according to claim 1 or 2, wherein the process is carried out in the presence of at least one base selected from the group consisting of KOtBu, NaOtBu and LiOtBu.

6. Process according to any one of the preceding claims, wherein the following catalysts of formula (III)
[M(L)(X)ₐ(L')_{b}] (III)
wherein
M is a transition metal chosen from the group consisting of Os, Co, Ru and Fe, and
X is a halogen anion, a carboxylate, borohydride, hydride, BF₄⁻ or PF₆⁻, and
L' is a monodentate phosphine ligand, and
L is a tridentate ligand of formula (IV) 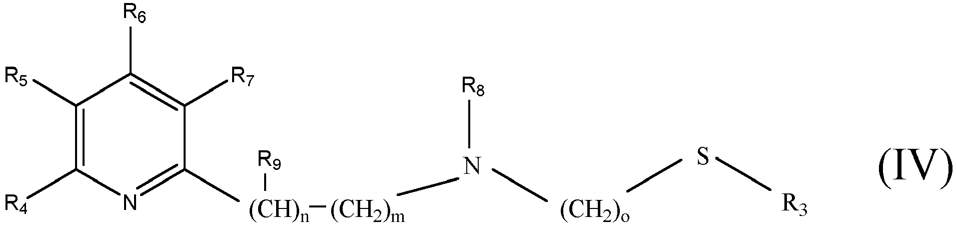 wherein
R₃ is -CH₃ or -CH₂CH₃, and
R₄ is H; -CH₃; -CH₂CH₃; -OCH₃ or -OCH₂CH₃, and
R₅ is H; -CH₃; -CH₂CH₃; -OCH₃ or -OCH₂CH₃,
or R₄ and R₅ form a C₄-C₈ ring system, which can be aliphatic or aromatic, and R₆ is H; -CH₃; -CH₂CH₃; -OCH₃ or -OCH₂CH₃, and
R₇ is H; -CH₃; -CH₂CH₃; -OCH₃ or -OCH₂CH₃, and
R₈ is H; -CH₃ or -CH₂CH₃, and
R₉ is -CH₃ or -CH₂CH₃, and
m is 0, 1 or 2, and
n is 0, 1 or 2,
with the proviso that the sum of m+n is 1 or 2,
o is 2 or 3,
a is 0, 1, 2, or 3,
b is 0, 1, 2, or 3,
with the proviso that the sum of a+b is 2 or 3, are used.

7. Process according to anyone of the preceding claims, wherein the following catalysts of formula (III')
M(L)(X)₂(L') **(III')**.
wherein
M is Ru or Fe, and
X is Cl⁻, and
L' is PPh₃, and
L is a tridentate ligand chosen from the group consisting of the ligands of formulae (IVa) - (IVI) 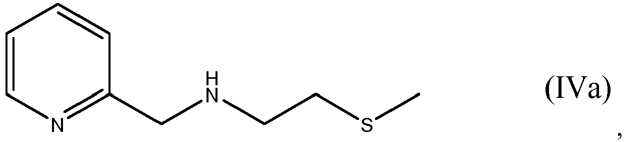 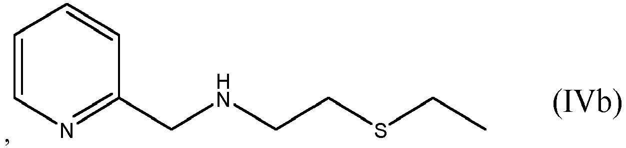 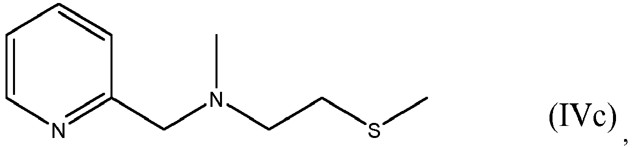 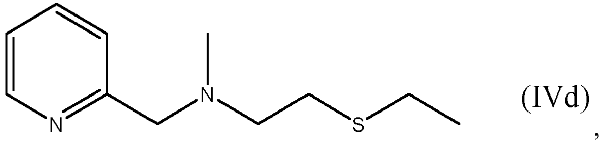 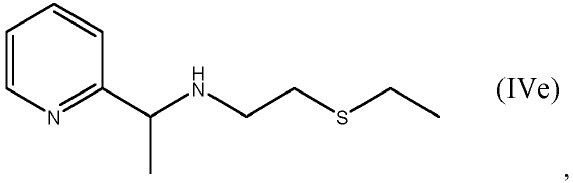 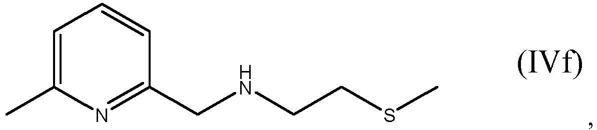 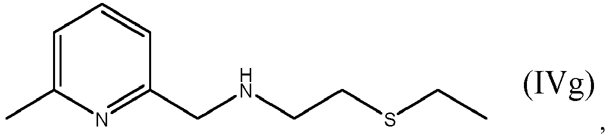 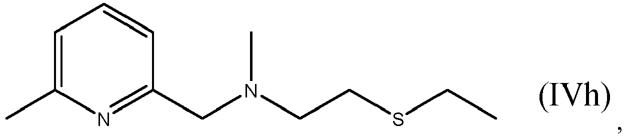 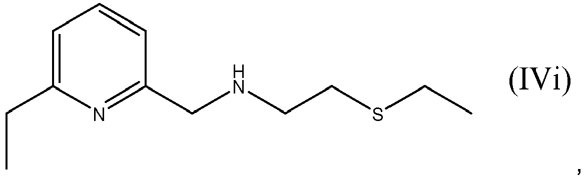 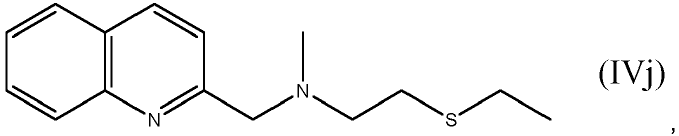 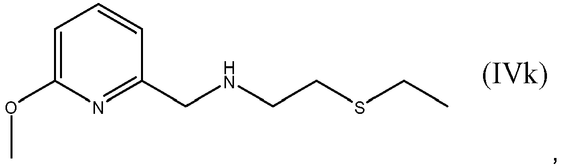 and 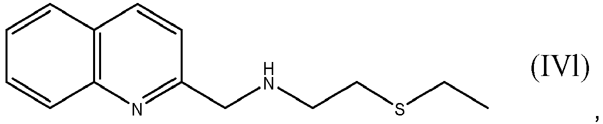 are used.

8. Process according to anyone of the preceding claims, wherein
the catalyst of formula (III) is used in an amount of 0.001 - 0.5 mol-% (based on the number of moles of the compounds of formula (I)).

9. Process according to anyone of the preceding claims, wherein the reduction is a transfer hydrogenation.

10. Process according to anyone of the preceding claims 1 - 8, wherein the process is carried out with H₂ gas.

11. Process according to claim 10, wherein the process is carried out at a pressure of 10 - 50bar.

12. Process according to anyone of the preceding claims, wherein the process is carried out at a temperature of 30 - 150 °C.

## Patentansprüche

1. Verfahren zur Herstellung einer Verbindung der Formel (II) 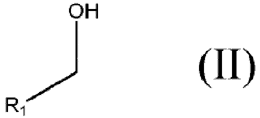 wobei
R₁ für ein aromatisches Ringsystem, das unsubstituiert ist, oder ein aromatisches Ringsystem, das substituiert ist, ein heteroaromatisches Ringsystem, das unsubstituiert ist, oder ein heteroaromatisches Ringsystem, das substituiert ist, ein aliphatisches Ringsystem, das unsubstituiert ist, oder ein aliphatisches Ringsystem, das substituiert ist, -CH₃, -CH₂CH₃, eine unsubstituierte C₃-C₂₂-Alkylgruppe, die linear oder verzweigt sein kann und auch teilweise ungesättigt sein kann, oder eine substituierte C₂-C₂₂-Alkylgruppe, die linear oder verzweigt sein kann und auch teilweise ungesättigt sein kann, steht,
durch selektive Reduktion einer Verbindung der Formel (I) 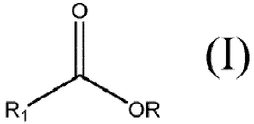 wobei
R für eine lineare C₁-C₆-Alkylgruppe, die substituiert sein kann, eine verzweigte C₃-C₆-Alkylgruppe, die substituiert sein kann, oder eine Benzylgruppe, die substituiert sein kann, steht und
R₁ für ein aromatisches Ringsystem, das unsubstituiert ist, oder ein aromatisches Ringsystem, das substituiert ist, ein heteroaromatisches Ringsystem, das unsubstituiert ist, oder ein heteroaromatisches Ringsystem, das substituiert ist, ein aliphatisches Ringsystem, das unsubstituiert ist, oder ein aliphatisches Ringsystem, das substituiert ist, -CH₃, -CH₂CH₃, eine unsubstituierte C₃-C₂₂-Alkylgruppe, die linear oder verzweigt sein kann und auch teilweise ungesättigt sein kann, oder eine substituierte C₂-C₂₂-Alkylgruppe, die linear oder verzweigt sein kann und auch teilweise ungesättigt sein kann, steht,
oder R und R₁ zusammen ein 4- bis 7-gliedriges Ringsystem, das substituiert sein kann, bilden, **dadurch gekennzeichnet, dass** die selektive Reduktion in Gegenwart mindestens eines Übergangsmetallkatalysators der Formel (III)
**[M(L)(X)ₐ(L')_{b}]** **(III)**
durchgeführt wird, wobei
M für ein Übergangsmetall steht und
X für ein Anion steht und
L' für einen einzähnigen Liganden steht und
L für einen dreizähnige Liganden der Formel (IV) 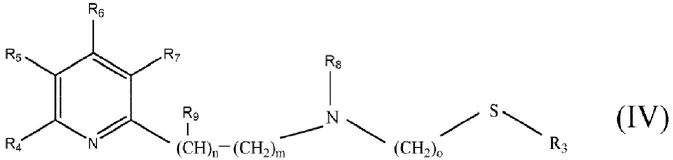 steht, wobei
R₃ für eine lineare C₁-C₄-Alkylgruppe, die substituiert sein kann, eine verzweigte C₃-C₄-Alkylgruppe, die substituiert sein kann, oder eine Phenylgruppe, die substituiert sein kann, steht und
R₄ für H, eine lineare C₁-C₄-Alkylgruppe, die substituiert sein kann, eine verzweigte C₃-C₄-Alkylgruppe, die substituiert sein kann, oder O-C₁-C₂-Alkyl steht und
R₅ für H, eine lineare C₁-C₄-Alkylgruppe, die substituiert sein kann, eine verzweigte C₃-C₄-Alkylgruppe, die substituiert sein kann, oder O-C₁-C₂-Alkyl steht
oder R₄ und R₅ ein C₄-C₈-Ringsystem, das aliphatisch oder aromatisch sein kann, bilden und
R₆ für H, eine lineare C₁-C₄-Alkylgruppe, die substituiert sein kann, eine verzweigte C₃-C₄-Alkylgruppe, die substituiert sein kann, oder O-C₁-C₂-Alkyl steht und
R₇ für H, eine lineare C₁-C₄-Alkylgruppe, die substituiert sein kann, eine verzweigte C₃-C₄-Alkylgruppe, die substituiert sein kann, oder O-C₁-C₂-Alkyl steht und
R₈ für H, eine lineare C₁-C₄-Alkylgruppe, die substituiert sein kann, eine verzweigte C₃-C₄-Alkylgruppe, die substituiert sein kann, steht und
R₉ für -CH₃ oder -CH₂CH₃ steht und
m für 0, 1 oder 2 steht und
n für 0, 1 oder 2 steht,
mit der Maßgabe, dass die Summe von m + n 1 oder 2
beträgt,
o für 2 oder 3 steht,
a für 0, 1, 2 oder 3 steht,
b für 0, 1, 2 oder 3 steht,
mit der Maßgabe, dass die Summe von a + b 2, 3
oder 4 beträgt.

2. Verfahren nach Anspruch 1, wobei das Verfahren in Gegenwart mindestens einer Base durchgeführt wird.

3. Verfahren nach Anspruch 1 oder 2, wobei das Verfahren in Gegenwart mindestens einer Base der Formel (VIII)
M¹(O-C₁-C₅-Alkyl) (VIII)
durchgeführt wird, wobei
M¹ für ein Alkalimetall steht.

4. Verfahren nach Anspruch 1 oder 2, wobei das Verfahren in Gegenwart mindestens einer Base der Formel (VIII')
M¹(O-C₃-C₅-Alkyl) (VIII')
durchgeführt wird, wobei
M¹ für Li, Na oder K steht.

5. Verfahren nach Anspruch 1 oder 2, wobei das Verfahren in Gegenwart mindestens einer Base aus der Gruppe bestehend aus KOtBu, NaOtBu und LiOtBu durchgeführt wird.

6. Verfahren nach einem der vorhergehenden Ansprüche, wobei die folgenden Katalysatoren der Formel (III)
**[M(L)(X)ₐ(L')_{b}]** **(III)**
wobei
M für ein Übergangsmetall aus der Gruppe bestehend aus Os, Co, Ru und Fe steht und
X für ein Halogen-Anion, ein Carboxylat, Borhydrid, Hydrid, BF₄⁻ oder PF₆⁻ steht und
L' für einen einzähnigen Phosphinliganden steht
und
L für einen dreizähnigen Liganden der Formel (IV) 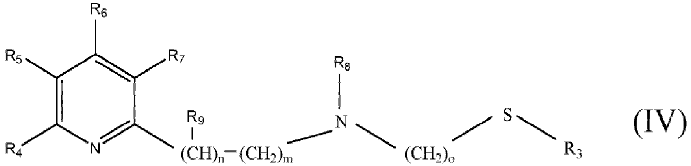 steht, wobei
R₃ für -CH₃ oder -CH₂CH₃ steht und
R₄ für H, -CH₃, -CH₂CH₃, -OCH₃ oder -OCH₂CH₃ steht und
R₅ für H, -CH₃, -CH₂CH₃, -OCH₃ oder -OCH₂CH₃ steht oder R₄ und R₅ ein C₄-C₈-Ringsystem, das aliphatisch oder aromatisch sein kann, bilden und
R₆ für H, -CH₃, -CH₂CH₃, -OCH₃ oder -OCH₂CH₃ steht und
R₇ für H, -CH₃, -CH₂CH₃, -OCH₃ oder -OCH₂CH₃ steht und
R₈ für H, -CH₃ oder -CH₂CH₃ steht und
R₉ für -CH₃ oder -CH₂CH₃ steht und
m für 0, 1 oder 2 steht und
n für 0, 1 oder 2 steht,
mit der Maßgabe, dass die Summe von m + n 1 oder 2 beträgt,
o für 2 oder 3 steht,
a für 0, 1, 2 oder 3 steht,
b für 0, 1, 2 oder 3 steht,
mit der Maßgabe, dass die Summe von a + b 2 oder 3 beträgt,
verwendet werden.

7. Verfahren nach einem der vorhergehenden Ansprüche, wobei die folgenden Katalysatoren der Formel (III')
**M(L)(X)₂(L')** **(III')**
wobei
M für Ru oder Fe steht und
X für Cl⁻ steht und
L' für PPh₃ steht und
L für einen dreizähnigen Liganden aus der Gruppe bestehend aus den Liganden der Formeln (IVa)-(IVl) 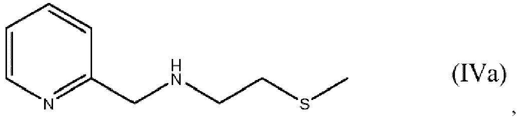 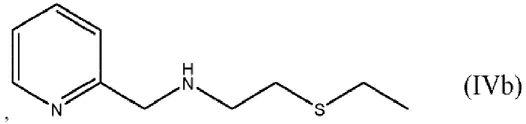 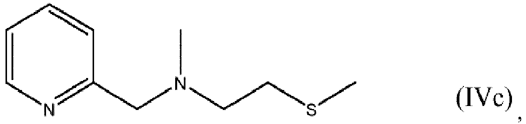 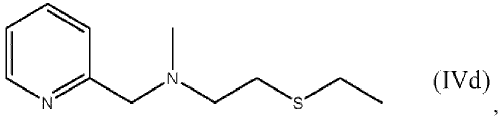 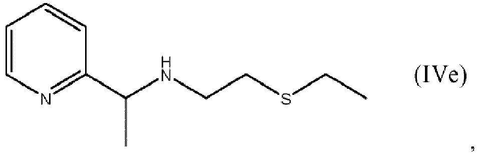 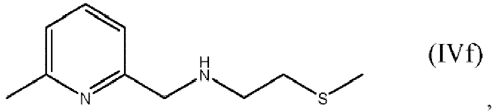 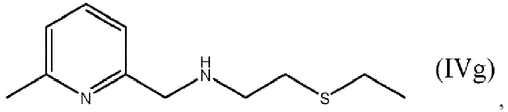 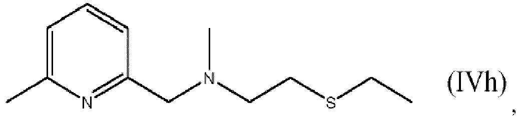 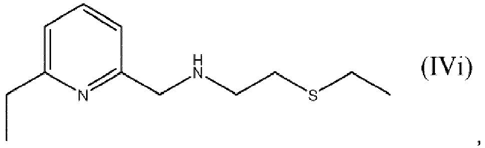 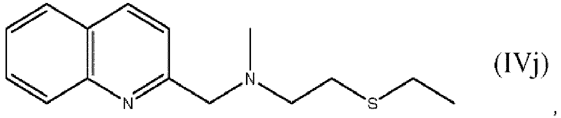 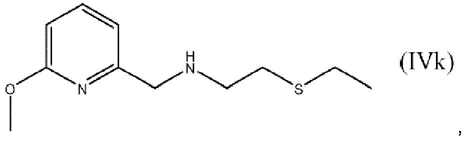 und 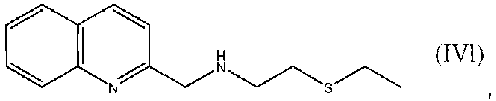 steht,
verwendet werden.

8. Verfahren nach einem der vorhergehenden Ansprüche, wobei der Katalysator der Formel (III) in einer Menge von 0,001-0,5 Mol-% (bezogen auf die Zahl der Mole der Verbindungen der Formel (I)) verwendet wird.

9. Verfahren nach einem der vorhergehenden Ansprüche, wobei es sich bei der Reduktion um eine Transferhydrierung handelt.

10. Verfahren nach einem der vorhergehenden Ansprüche 1-8, wobei das Verfahren mit H₂-Gas durchgeführt wird.

11. Verfahren nach Anspruch 10, wobei das Verfahren bei einem Druck von 10-50 bar durchgeführt wird.

12. Verfahren nach einem der vorhergehenden Ansprüche, wobei das Verfahren bei einer Temperatur von 30-150 °C durchgeführt wird.

## Revendications

1. Procédé de production d'un composé de formule (II) 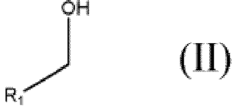
R₁ étant un système cyclique aromatique qui est non substitué ou un système cyclique aromatique qui est substitué ; un système cyclique hétéroaromatique qui est non substitué ou un système cyclique hétéroaromatique qui est substitué ; un système cyclique aliphatique qui est non substitué ou un système cyclique aliphatique qui est substitué ; -CH₃ ; -CH₂CH₃ ; un groupe C₃₋₂₂-alkyle non substitué, qui peut être linéaire ou ramifié et qui peut également être partiellement insaturé ; ou un groupe C₂₋₂₂-alkyle substitué, qui peut être linéaire ou ramifié et qui peut également être partiellement insaturé
par une réduction sélective d'un composé de formule (I) 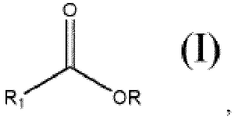
R étant un groupe C₁₋₆-alkyle linéaire, qui peut être substitué ; un groupe C₃₋₆-alkyle ramifié, qui peut être substitué ou un groupe benzyle, qui peut être substitué, et
R₁ étant un système cyclique aromatique qui est non substitué ou un système cyclique aromatique qui est substitué ; un système cyclique hétéroaromatique qui est non substitué ou un système cyclique hétéroaromatique qui est substitué ; un système cyclique aliphatique qui est non substitué ou un système cyclique aliphatique qui est substitué ; -CH₃ ; -CH₂CH₃ ; un groupe C₃₋₂₂-alkyle non substitué, qui peut être linéaire ou ramifié et qui peut également être partiellement insaturé ; ou un groupe C₂₋₂₂-alkyle substitué, qui peut être linéaire ou ramifié et qui peut également être partiellement insaturé,
ou R et R₁ formant ensemble un système cyclique à 4 à 7 chaînons, qui peut être substitué,
**caractérisé en ce que** la réduction sélective est mise en œuvre en présence d'au moins un catalyseur de métal de transition de formule (III)
**[M(L)(X)ₐ(L')_{b}]** **(III),**
M étant un métal de transition et
X étant un anion, et
L' étant un ligand monodentate, et
L étant un ligand tridentate de formule (IV) 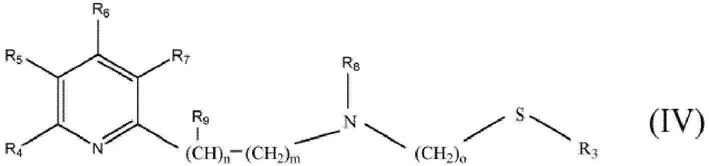
R₃ étant un groupe C₁₋₄-alkyle linéaire, qui peut être substitué ; un groupe C₃₋₄-alkyle ramifié, qui peut être substitué ; ou un groupe phényle, qui peut être substitué, et
R₄ étant H ; un groupe C₁₋₄-alkyle linéaire, qui peut être substitué ; un groupe C₃₋₄-alkyle ramifié, qui peut être substitué ; ou OC₁₋₂-alkyle, et
R₅ étant H ; un groupe C₁₋₄-alkyle linéaire, qui peut être substitué ; un groupe C₃₋₄-alkyle ramifié, qui peut être substitué ; ou OC₁₋₂-alkyle,
ou R₄ et R₅ formant un système cyclique en C₄₋₈, qui peut être aliphatique ou aromatique, et
R₆ étant H ; un groupe C₁₋₄-alkyle linéaire, qui peut être substitué ; un groupe C₃₋₄-alkyle ramifié, qui peut être substitué ; ou OC₁₋₂-alkyle, et
R₇ étant H ; un groupe C₁₋₄-alkyle linéaire, qui peut être substitué ; un groupe C₃₋₄-alkyle ramifié, qui peut être substitué ; ou OC₁₋₂-alkyle, et
R₈ étant H ou un groupe C₁₋₄-alkyle linéaire, qui peut être substitué ; un groupe C₃₋₄-alkyle ramifié, qui peut être substitué, et
R⁹ étant -CH₃ ou -CH₂CH₃, et
m étant 0, 1 ou 2, et
n étant 0, 1 ou 2,
étant entendu que la somme m + n est 1 ou 2,
o étant 2 ou 3,
a étant 0, 1, 2, ou 3,
b étant 0, 1, 2, ou 3,
étant entendu que la somme a + b est 2, 3 ou 4.

2. Procédé selon la revendication 1, le procédé étant mis en œuvre en présence d'au moins une base.

3. Procédé selon la revendication 1 ou 2, le procédé étant mis en œuvre en présence d'au moins une base de formule (VIII)
**M¹(OC₁-C₅alkyl)** **(VIII),**
M¹ étant un métal alcalin.

4. Procédé selon la revendication 1 ou 2, le procédé étant mis en œuvre en présence d'au moins une base de formule (VIII'),
**M¹(OC₃-C₅alkyl)** **(VIII')**
M¹ étant Li, Na ou K.

5. Procédé selon la revendication 1 ou 2, le procédé étant mis en œuvre en présence d'au moins une base choisie dans le groupe constitué par KOtBu, NaOtBu et LiOtBu.

6. Procédé selon l'une quelconque des revendications précédentes, les catalyseurs suivants de formule (III)
**[M(L)(X)ₐ(L')_{b}]** **(III).**
M étant un métal de transition choisi dans le groupe constitué par Os, Co, Ru et Fe, et
X étant un anion d'halogène, un carboxylate, un borohydrure, un hydrure, BF₄⁻, ou PF₆⁻, et
L' étant un ligand phosphine monodentate, et
L étant un ligand tridentate de formule (IV) 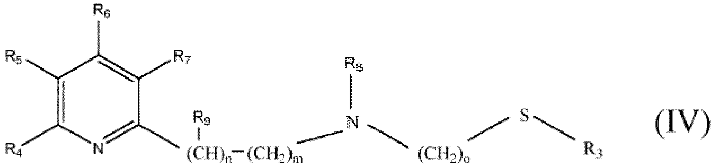
R₃ étant -CH₃ ou -CH₂CH₃, et
R₄ étant H ; -CH₃ ; -CH₂CH₃ ; -OCH₃ ou -OCH₂CH₃, et R₅ étant H ; -CH₃ ; -CH₂CH₃ ; -OCH₃ ou -OCH₂CH₃,
ou R₄ et R₅ formant un système cyclique en C₄₋₈, qui peut être aliphatique ou aromatique, et
R₆ étant H ; -CH₃ ; -CH₂CH₃ ; -OCH₃ ou -OCH₂CH₃, et
R₇ étant H ; -CH₃ ; -CH₂CH₃ ; -OCH₃ ou -OCH₂CH₃, et
R₈ étant H ; -CH₃ ou -CH₂CH₃, et
R⁹ étant -CH₃ ou -CH₂CH₃, et
m étant 0, 1 ou 2, et
n étant 0, 1 ou 2,
étant entendu que la somme m + n est 1 ou 2,
o étant 2 ou 3,
a étant 0, 1, 2, ou 3,
b étant 0, 1, 2, ou 3,
étant entendu que la somme a + b est 2, ou 3, étant utilisés.

7. Procédé selon l'une quelconque des revendications précédentes, les catalyseurs suivants de formule (III')
**M(L)(X)₂(L')** **(III)**,
M étant Ru ou Fe, et
X étant Cl⁻ ; et
L' étant PPh₃, et
L étant un ligand tridentate choisi dans le groupe constitué par les ligands des formules (IVa) à (IVl) 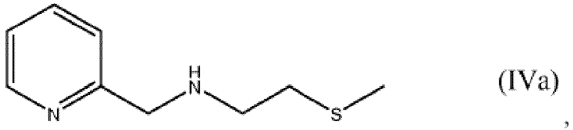 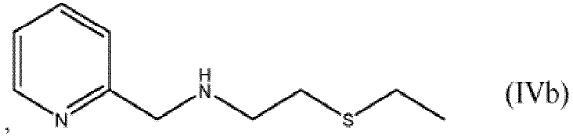 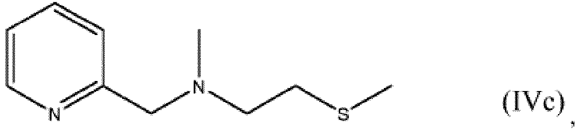 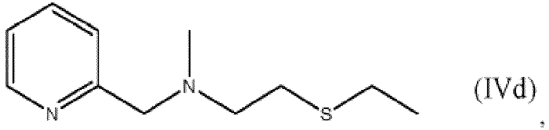 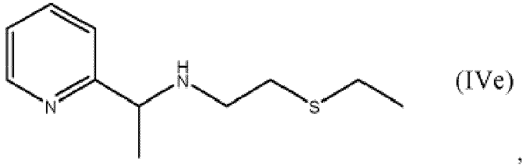 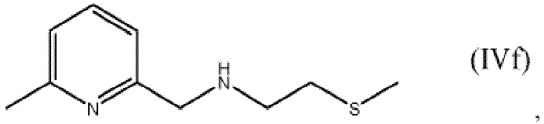 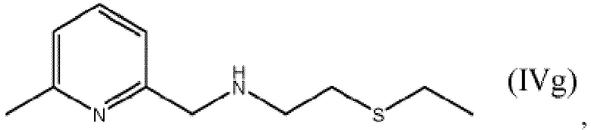 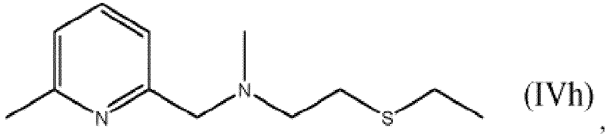 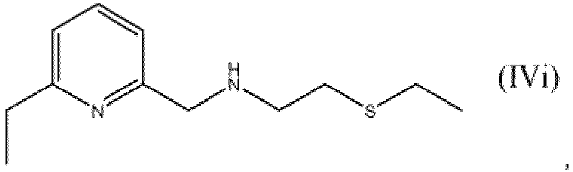 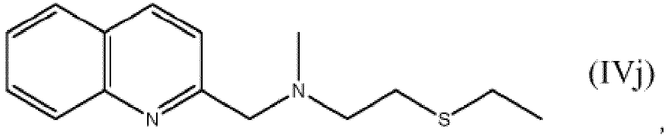 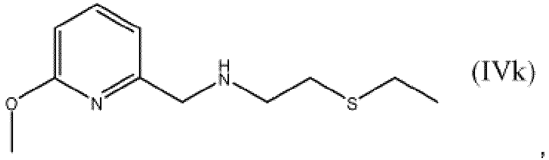 et 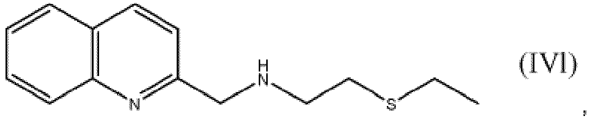 étant utilisés.

8. Procédé selon l'une quelconque des revendications précédentes, le catalyseur de formule (III) étant utilisé en une quantité de 0,001 à 0,5 % en moles (sur la base du nombre de moles des composés de formule (I)).

9. Procédé selon l'une quelconque des revendications précédentes, la réduction étant une hydrogénation par transfert.

10. Procédé selon l'une quelconque des revendications précédentes 1 à 8, le procédé étant mis en œuvre avec du gaz H₂.

11. Procédé selon la revendication 10, le procédé étant mis en œuvre à une pression de 10 à 50 bars.

12. Procédé selon l'une quelconque des revendications précédentes, le procédé étant mis en œuvre à une température de 30 à 150 °C.
